# EUROPEAN PATENT SPECIFICATION

(11) **EP 3 474 915 B1**
(45) Date of publication and mention of the grant of the patent: **24.02.2021**
(21) Application number: 17737950.0
(22) Date of filing: 23.06.2017
(51) Int. Cl.: A61L 29/08, A61L 29/14, A61L 29/16

(54) **DRUG COATED BALLOONS**
ARZNEIMITTELBESCHICHTETER BALLON
BALLONNETS REVÊTUS DE MÉDICAMENT

(30) Priority: 24.06.2016 US 201662354636 P; 28.10.2016 US 201662414459 P; 11.11.2016 US 201662421112 P; 23.12.2016 US 201662438547 P; 27.12.2016 US 201662439351 P; 21.04.2017 US 201762488646 P
(43) Date of publication of application: 01.05.2019
(73) Proprietor: W. L. Gore & Associates, Inc., Newark, DE 19711 (US)
(72) Inventor: Alston, Steven M., Newark, Delaware 19711 (US); Edelman, Elazer, Cambridge, Massachusetts 02139 (US); Heicksen, Peter, Newark, Delaware 19711 (US); Holland, Theresa, Newark, Delaware 19711 (US); Markham, Peter, Lexington, Massachusetts 02421 (US); Traylor, Peter, Newark, Delaware 19711 (US); Tzafriri, Abraham, Lexington, Massachusetts 02421 (US); Zani, Brett G., Lexington, Massachusetts 02421 (US)
(74) Representative: HGF
(86) International application number: PCT/US2017/039143
(87) International publication number: WO 2017/223536

(56) References cited:
- WO-A1-2016/061186
- US-A1- 2014 271 775
- US-A1- 2014 358 122

## Description

### CROSS-REFERENCE TO RELATED APPLICATIONS

The present application claims priority and benefit from U.S. Provisional Application No. 62/354,636, filed June 24, 2016, entitled "FLUOROPOLYMER BALLOON CATHETER DEVICE AND METHOD FOR INCREASING ARTERIAL PERMEABILITY AND DRUG RETENTION," U.S. Provisional Application No. 62/414,459, filed October 28, 2016, entitled "FLUOROPOLYMER BALLOON CATHETER DEVICE AND METHOD FOR INCREASING ARTERIAL PERMEABILITY AND DRUG RETENTION," U.S. Provisional Application No. 62/421,112, filed November 11, 2016, entitled "FLUOROPOLYMER BALLOON CATHETER DEVICE AND METHOD FOR INCREASING ARTERIAL PERMEABILITY AND DRUG RETENTION," U.S. Provisional Application No. 62/438,547, filed December 23, 2016, entitled "FLUOROPOLYMER BALLOON CATHETER DEVICE AND METHOD FOR INCREASING ARTERIAL PERMEABILITY AND DRUG RETENTION," U.S. Provisional Application No. 62/439,351, filed December 27, 2016, entitled "FLUOROPOLYMER BALLOON CATHETER DEVICE AND METHOD FOR INCREASING ARTERIAL PERMEABILITY AND DRUG RETENTION," and U.S. Provisional Application No. 62/488,646 filed April 21, 2017, entitled "FLUOROPOLYMER BALLOON CATHETER DEVICE AND METHOD FOR INCREASING ARTERIAL PERMEABILITY AND DRUG RETENTION".

### FIELD OF THE INVENTION

The present invention relates to drug coated balloons, and in particular to drug coated balloons having a microcrystalline structure. Also disclosed herein are techniques for increasing vascular permeability for drug application and retention.

### BACKGROUND OF THE INVENTION

Vascular diseases, such as atherosclerosis, artery occlusion, and restenosis, are a leading cause of human mortality and morbidity. Vascular diseases arise from a variety of causes, and in some cases, necessitate surgical or endovascular intervention. Trauma to the vascular system can also necessitate surgical intervention to treat the traumatized anatomy. The treatment of vascular disease at a local, rather than systemic, level is often preferred. Systemic administration of drugs can produce unwanted side effects, when compared to the local administration of a drug to a target tissue to treat vascular disease. The utilization of a drug-coated endovascular medical device has become a standard technique in the treatment of vascular disease. In particular, a common treatment for vascular disease is the short-term or long-term contact of a tissue with an endovascular medical device, such as a balloon or a stent, respectively, that is coated with a drug that prevents or reduces vascular disease at the site of contact. The short-term contact of vascular medical devices including catheter-based balloons, are often undertaken to treat vascular diseases and vascular trauma, and the long-term contact, e.g., implantation, of endovascular medical devices including vascular grafts, stent-grafts, and stents. Upon contact of the endovascular medical device with a diseased vascular tissue, the drug elutes from the endovascular medical device into the surrounding tissue at the site of contact, thereby treating the vascular disease at a local, rather than systemic, level.

Drug coated balloons (DCBs) are one example of a drug-coated endovascular medical device. The literature discloses the use of DCBs for the treatment of vascular diseases, including coronary artery disease and peripheral artery disease (see e.g., U.S. Pat. No. 5,102,402 to Dror et al.). Dror *et al.* disclose placing a DCB in a blood vessel lumen to treat the vessel wall, inflating the balloon, and contacting the balloon surface with the luminal vessel wall to deliver a drug into the blood vessel wall. The dosing of the drug to the treatment site using DCBs can be highly variable and unpredictable immediately after implantation or deployment, and local drug levels in the vascular tissue can be highly variable and unpredictable over an extended time. It is therefore desirable to have improved drug coated balloons and techniques for treating vascular disease that are reliable and reproducible in drug dosing.

In order to improve upon conventional DCBs and techniques for treating vascular disease, DCBs may be constructed of one or more layers of material selected to provide certain properties to optimize performance of the DCB in some particular way, depending on the application. In the case of multi-layer or composite balloons, the multiple layers within the composite may be different materials to obtain a blend of physical and/or chemical properties to optimize performance. U.S. Patent Application Publication No. 2016/0106961 to Cully et al. discloses composite or layered DCBs. The described composite materials can comprise a porous layer adhered to a blow moldable polymer, such as a composite material that comprises an expanded fluoropolymer layer that is adhered to a blow moldable polymer through a stretch blow molding process. Additionally, U.S. Patent Application Publication No. 2014/0271775 to Cleek et al. discloses composite or layered DCBs comprising substrates having oriented drug crystals of high aspect ratio habit. The described composite materials can comprise a substrate comprising a porous microstructure and an amount of crystalline paclitaxel comprising hollow crystal habits associated with the substrate.

Although the multi-layer or composite balloons described in Cully *et al.* and Cleek *et al.* achieve some success in the reliability and reproducibility of drug dosing, many of the conventional multi-layer or composite balloons demonstrate lower than optimal drug application and retention. This phenomenon arises due to a number of factors including a non-uniform coating of the drug on the substrate, particulation or loss of the drug coating during deployment of the DCB, and/or a non-uniform or incomplete transfer of the drug coating to the surface of the vessel lumen, which ultimately results in lower than optimal drug application and retention. Accordingly, the need exists for improved drug coated balloons and techniques for increasing vascular permeability for drug application and retention.

### SUMMARY OF THE INVENTION

The scope of this invention is defined by the claims. Embodiments in the description relating to methods of treatment are not covered by the claims. Any "embodiment" or "example" which is disclosed in the description but is not covered by the claims should be considered as presented for illustrative purpose only.

It has been thought that local tissue retention determines biological responses, but little has been understood regarding factors that determine retention in balloon based delivery. The coating morphology (e.g., the drug coating on at least a portion of the surface of balloon) versus drug-tissue binding was investigated using various methods to visualize the coating morphology and arterial drug distribution and retention. Imaging of various balloon surfaces coated with the different drug formulations comprising similar paclitaxel doses (e.g., 3.5 µg/mm²) revealed different coating morphologies. Imaging of various balloon surface coated with the same drug formulations comprising similar paclitaxel doses (e.g., 3.5 µg/mm²) revealed similar coating morphologies. In particular, currently available commercial drug coated balloons were compared versus various drug coated balloon embodiments described herein, and it was found that the commercial drug coated balloons had a fractured, amorphous morphology and the various drug coated balloon embodiments described herein had a microcrystalline morphology. Imaging of treated surfaces revealed differential coating efficiencies that correlated with differential drug delivery and retention where the various drug coated balloon embodiments described herein provided better delivery and retention.

Additionally, balloons with either a porous microstructure or a nonporous microstructure were investigated for utilization as drug-coated endovascular medical devices. In particular, it has been thought that increased drug dose or number of drug-coated balloon (DCB) inflations increases delivery and tissue retention of drug; however, this has never been validated. Upon further examination, it was surprisingly discovered that utilization of DCBs to provide multiple treatments at a site resulted in tissue retention of the drug that was higher than would be expected by the dose exposure alone. The multiple treatments may be provided: (i) using multiple DCBs, each inflated one or more times, or (ii) using a single DCB that is inflated a plurality of times.

In various embodiments, a medical device is provided for that includes a balloon including an outer surface, and a drug coating layer on the outer surface of the balloon. The drug coating layer includes microcrystals in a haystack orientation. Optionally, the microcrystals have a random and a substantial absence of uniformity in angles from the outer surface of the balloon. The drug coating may include paclitaxel.

In some embodiments, the outer surface of the balloon includes a non-porous polymer. In other embodiments, the outer surface of the balloon includes a porous polymer.

In certain embodiments, the balloon includes a layer material, wherein the layered material comprises a polymer layer adhered to a fluoropolymer layer including a porous microstructure, wherein layers are in overlying relationship to each other and the fluoropolymer layer is an outermost layer. Optionally, the drug coating layer penetrates the outer surface of the balloon by an average penetration depth of from 2 to 10 µm.

In various embodiments, a medical balloon is provided for that includes a thermoplastic polymeric layer defining an interior chamber, a polymeric layer over at least a portion of the thermoplastic polymeric layer, and a coating layer on at least a portion of the polymeric layer. The coating layer includes a therapeutic agent, an excipient, and microcrystals in a haystack orientation having random and a substantial absence of uniformity in placement on an outer surface of the polymeric layer.

In some embodiments, the polymeric layer is porous. In other embodiments, the polymeric layer is non-porous.

In some embodiments, a majority of the microcrystals each have a major dimension length that is at least 10 times greater than a major dimension width of the microcrystal.

In some embodiments, the microcrystals have a random and a substantial absence of uniformity in angles from the outer surface of the polymeric layer, and a majority of the microcrystals project from the outer surface at an angle of 5° to 15°.

In certain embodiments, the therapeutic agent includes paclitaxel, docetaxel, protaxel, arsenic trioxide, thalidomide, atorvastatin, cerivastatin, fluvastatin, betamethasone diproprionate, dexamethasone 21-palmitate, sirolimus, everolimus, zotarolimus, biolimus or temsirolimus.

In some embodiments, the coating layer includes the therapeutic agent and the excipient in a predetermined weight ratio of between 3:1 and 20:1. Optionally, a majority of the microcrystals each have a major dimension length that is at least 13 or at least 15 times a major dimension width. In other embodiments, a majority of the microcrystals each have a major dimension length that is between 12 µm and 22 µm, and the majority of the microcrystals each have a major dimension width that is between 0.5 µm and 2 µm.

In some embodiments, when the medical balloon is inflated in a vessel lumen for one minute, at least a portion of the coating layer transfers to at least a portion of the vessel lumen such that one hour after the inflation at least 14% of the portion of the vessel lumen is covered by the coating layer.

In other embodiments, when the medical balloon is inflated in a vessel lumen for one minute, at least a portion of the coating layer transfers to at least a portion of the vessel lumen such that one hour after the inflation at least 12% of a portion of a surface of the vessel lumen is covered by the coating layer.

In some embodiments, when the medical balloon is inflated in a vessel lumen for one minute, at least a portion of the coating layer uniformly transfers to the vessel lumen along a length of the vessel lumen.

Also disclosed herein is a method for preparing a tissue for drug application whereby tissue retention is improved. The method includes providing one or more medical devices including a balloon including an outer surface and a drug coating on the outer surface of the balloon, and sequentially treating a vascular treatment site n times with the one or more medical devices. The dose amount of a drug from the drug coating that is retained by the tissue at one hour after the sequential treating is greater than the n times a dose amount retained by the tissue at one hour after a single treatment.

In some embodiments, the dose amount retained in the tissue at the one hour after the sequential treatment is about six times the dose amount retained by the tissue at the one hour after the single treatment, where the dose amount retained by the tissue at the one hour after the single treatment is from 1% to 10% of a loaded dose of the drug coating.

Optionally, a half-life of the drug retained in the tissue after the sequential treatment is greater than 13 hours. In some embodiments, the balloon is configured to release from 65% to 85% of drug from the drug coating upon inflation.

In certain embodiments, a dose amount of the drug retained in the tissue at the one hour after the sequentially treating is greater than 750 µg/g. In other embodiments, a dose amount of the drug retained in the tissue at the one hour after the sequentially treating is greater than 1150 µg/g. In other embodiments, the dose amount of the drug retained in the tissue at the one hour after the sequentially treating is greater than 50 µg/g. In yet other embodiments, the dose amount of the drug retained in the tissue at the one day after the sequentially treating is greater than 1 µg/g.

Also disclosed herein is a method for preparing a vessel for drug application whereby tissue retention is improved. The method includes providing a medical device comprising a balloon comprising an outer surface and a drug coating on the outer surface of the balloon, where the drug coating comprises microcrystals in a haystack orientation having random and a substantial absence of uniformity in placement on the outer surface of the balloon, and treating a vascular treatment site with the medical device.

In some embodiments, the drug coating includes paclitaxel, the treating includes inflating the balloon at the treatment site for 1 minute, and when the balloon is inflated at the treatment site for 1 minute, less than about 35% of the drug coating remains on the outer surface of the balloon.

In some embodiments, the balloon includes a porous material, and where the drug coating includes paclitaxel, the treating includes inflating the balloon at the treatment site for 1 minute, and when the balloon is inflated at the treatment site for 1 minute, between about 15% and about 30% of the drug coating remains on the outer surface of the balloon. In other embodiments, the balloon includes a non-porous material, and where the drug coating includes paclitaxel, the treating includes inflating the balloon at the treatment site for 1 minute, and when the balloon is inflated at the treatment site for 1 minute, less than about 10% of the drug coating remains on the outer surface of the balloon.

In certain embodiments, the drug coating includes paclitaxel at a dose of 3-4 µg/mm². Optionally, a dose amount of a drug from the drug coating retained in the tissue at one hour after the treating is greater than 5% of a load dose amount on the balloon. In some embodiments, the balloon includes a porous material, and a dose amount of a drug from the drug coating retained in the tissue at 72 hours after the treating is greater than 2% of a load dose amount on the balloon.

In some embodiments, the balloon includes a porous material, and a dose amount of a drug from the drug coating layer retained in the tissue at one hour after the treating is greater than 50 µg/g. In other embodiments, the balloon includes a non-porous material, and an enface tissue coating at one hour after the treating is greater than 12%. In yet other embodiments, the balloon includes a porous material, and an enface tissue coating at one hour after the treating is greater than 15%.

In various embodiments, a medical device is provided for that includes an elongate catheter shaft having a proximal section, a distal section, and an inflation lumen, and a balloon on the distal section of the shaft and including a balloon wall defining a chamber and including a layered material. The layered material may include a polymer layer adhered to a fluoropolymer layer comprising a porous microstructure, and the polymer layer and the fluoropolymer layer may be in an overlying relationship to each other and the fluoropolymer layer may be an outermost layer. The medical device further includes a drug coating on the fluoropolymer layer. The drug coating has microcrystals in a haystack orientation.

In some embodiments, the drug coating comprises paclitaxel and an excipient. Optionally, the drug coating comprises paclitaxel and the excipient in a predetermined weight ratio of between 3:1 and 20:1.

In certain embodiments, a dose density of the drug coating is from 2.0 to 7.0 µg/mm².

In some embodiments, the balloon is configured to release from 70% to 85% of a drug from the drug coating upon an inflation time of about 60 seconds.

In some embodiments, the microcrystals have a random and a substantial absence of uniformity in angles from the fluoropolymer layer, and a majority of the microcrystals project from the outer surface at an angle of 5° to 15°.

Optionally, the drug coating penetrates the fluoropolymer layer by an average penetration depth of from 2 to 10 µm.

### BRIEF DESCRIPTION OF THE DRAWINGS

The accompanying drawings are included to provide a further understanding of the invention and are incorporated in and constitute a part of this specification, illustrate embodiments of the invention, and together with the description serve to explain the principles of the invention.
FIG. 1 shows the assembly of a microtubule by polymerization in accordance with some aspects of the present disclosure.
FIGS. 2A, 2B, and 2C show micrographs of a comparative DCB and a DCB embodiment in accordance with some aspects of the present disclosure.
FIG. 3A is a graph of a comparison of tissue retention time of a drug delivered by three different commercial DCBs.
FIG. 3B is a table of a comparison of biological effects of a drug delivered by three different commercial DCBs.
FIG. 4A shows a micrograph of a comparative DCB in accordance with some aspects of the present disclosure.
FIG. 4B shows particulation from a comparative DCB in accordance with some aspects of the present disclosure.
FIG. 5A shows a micrograph of a DCB embodiment in accordance with some aspects of the present disclosure.
FIG. 5B shows a DCB embodiment substantially devoid of particulation in accordance with some aspects of the present disclosure.
FIG. 6A shows a micrograph comparison of a peripheral artery following drug delivery by different DCBs tested in Example 3 in accordance with some aspects of the present disclosure.
FIG. 6B is an enface tissue coating comparison of a drug delivered by different DCBs tested in Example 3 in accordance with some aspects of the present disclosure.
FIG. 7A shows a medical balloon embodiment in accordance with some aspects of the present disclosure.
FIG. 7B shows a cross-section of composite material forming the DCB embodiment shown in FIG. 7A.
FIG. 7C shows a cross-section of composite material forming the DCB embodiment shown in FIG. 7A.
FIG. 7D shows a thickness characterization of a composite material forming a DCB in accordance with some aspects of the present disclosure.
FIGS. 8A and 8B show microcrystalline morphology on non-porous and porous balloon substrates in accordance with some aspects of the present disclosure.
FIG. 9 shows a balloon catheter assembly in accordance with some aspects of the present disclosure.
FIG. 10 is a graph of total arterial paclitaxel content following 1 time or 3 time inflations of DCBs tested in Example 1 in accordance with some aspects of the present disclosure.
FIG. 11A is a graph of day 30 standard histomorphology parameters following 1 time or 3 time inflations of DCBs tested in Example 1 in accordance with some aspects of the present disclosure.
FIG. 11B shows histologic specimens of an artery following 1 time or 3 time inflations of DCBs tested in Example 1 in accordance with some aspects of the present disclosure.
FIG. 12 is a graph of a seven day scanning electron microscopy (SEM) assessment following 1 time or 3 time inflations of DCBs tested in Example 1 in accordance with some aspects of the present disclosure.
FIG. 13 is a drug release comparison of different DCBs tested in Example 3 in accordance with some aspects of the present disclosure.
FIG. 14A is a graph of a comparison of tissue retention of a drug delivered by different DCBs tested in Example 2 in accordance with some aspects of the present disclosure.
FIG. 14B is a table of a comparison of tissue retention of a drug delivered by different DCBs tested in Example 2 in accordance with some aspects of the present disclosure.
FIG. 14C is a tissue retention comparison of a drug delivered by different DCBs tested in Example 2 in accordance with some aspects of the present disclosure.
FIG. 15 is a drug release comparison of different DCBs tested in Example 2 in accordance with some aspects of the present disclosure.
FIG. 16.is a comparison of tissue retention of a drug delivered by different DCBs tested in Example 2 in accordance with some aspects of the present disclosure.
FIG. 17 is a comparison of tissue retention of a drug delivered by different DCBs tested in Example 3 in accordance with some aspects of the present disclosure.
FIG. 18 is a graph depicting the enface tissue coating of various DCBs tested in Example 3 in accordance with some aspects of the present disclosure.
FIG. 19 is a graph depicting the enface tissue coating of various DCBs tested in Example 3.
FIG. 20 is graph of a comparison of drug coating on different DCBs tested in Example 4 in accordance with some aspects of the present disclosure.
FIGS. 21A and 21B, and 21C are graphs of comparison of different DCBs after acute tissue transfer tested in Example 4 in accordance with some aspects of the present disclosure.
FIG. 22 is a graph of comparison of drug coating on different DCBs tested in Example 4 in accordance with some aspects of the present disclosure.
FIG. 23 is a graph of comparison of drug coating particulation from different DCBs tested in Example 4 in accordance with some aspects of the present disclosure.

### DETAILED DESCRIPTION OF THE ILLUSTRATED EMBODIMENTS

Persons skilled in the art will readily appreciate that various aspects of the present disclosure can be realized by any number of methods and apparatuses configured to perform the intended functions. That is, other methods and apparatuses can be incorporated herein to perform the intended functions. It should also be noted that the accompanying drawing figures referred to herein are not all drawn to scale, but may be exaggerated to illustrate various aspects of the present disclosure. The figures should not be construed as limiting. Finally, although the present disclosure may be described in connection with various principles and beliefs, the present disclosure should not be bound by theory.

The following terms as used herein mean:

The terms "a" and "an" are defined as one or more unless this disclosure explicitly requires otherwise.

The terms "substantially," "approximately" and "about" are defined as being largely but not necessarily wholly what is specified (and include wholly what is specified) as understood by one of ordinary skill in the art. In any disclosed embodiment, the term "substantially," "approximately," or "about" may be substituted with "within [a percentage] of' what is specified, where the percentage includes 0.1, 1, 5, and 10 percent. The term "majorly" or "majority" indicates at least half or 50%.

The terms "comprise" (and any form of comprise, such as "comprises" and "comprising"), "have" (and any form of have, such as "has" and "having"), "include" (and any form of include, such as "includes" and "including") and "contain" (and any form of contain, such as "contains" and "containing") are open-ended linking verbs. As a result, any of the present devices, systems, and methods that "comprises," "has," "includes" or "contains" one or more elements possesses those one or more elements, but is not limited to possessing only those one or more elements. Likewise, an element of a device, system, or method that "comprises," "has," "includes" or "contains" one or more features possesses those one or more features, but is not limited to possessing only those one or more features.

Furthermore, a structure that is capable of performing a function or that is configured in a certain way is capable or configured in at least that way, but may also be capable or configured in ways that are not listed.

As used herein, "nominal diameter" means the approximate diameter of the balloon at the nominal inflation pressure. Beyond this state, pressure increases (e.g., up to the rated burst pressure) result in less than a 20% increase in diameter, less than a 15% increase in diameter, or less than a 10% increase in diameter. Typically, the nominal diameter is the labeled diameter as indicated on the instructions for the end user, e.g., a clinician.

According to the claimed invention, a "microcrystal haystack" or the phrase "microcrystals in a haystack orientation" is defined as a plurality of microcrystals, the majority of which are oriented at less than a 20 degree angle from a surface on which they are positioned. In addition, the majority of the plurality of microcrystals each have a major (overall) dimension length that is at least five (5) times greater than the major (overall) dimension width of the microcrystal.

### I. Introduction

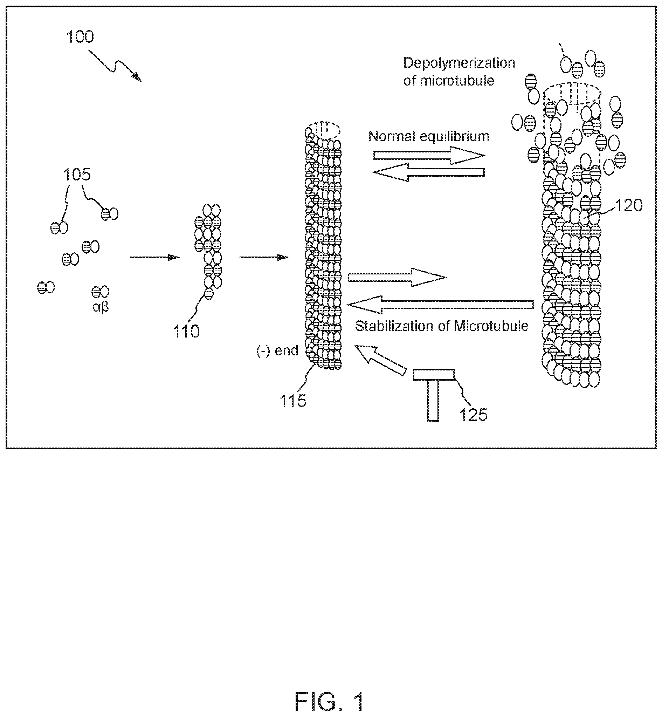

In various embodiments, a balloon is provided for that comprises an outer surface and a drug coating layer on the outer surface of the balloon. Conventional approaches for the treatment of vascular disease include the utilization of a drug coated balloon (DCB). However, a problem associated with traditional DCBs is the difficulty in maintaining tissue retention of the drug and reducing drug loss in the biological fluid, e.g., blood, during DCB transport to a therapeutic location, particularly when factors are present that increase rates at which drugs associate with and/or dissociate from receptors (i.e., reversible binding and binding kinetics). For example, the reversible binding of drugs to receptors within tissue is a function of dose and residence time. FIG. 1 shows how the reversible binding of a drug such as a taxane to receptor sites within a microtubule may affect tissue retention. The assembly 100 of a microtubule by the polymerization of αβ tubulin heterodimers 105 occurs in two phases: nucleation 110 and elongation 115. Formation of a short polymerization nucleus precedes elongation or polymer growth at each end by the reversible, noncovalent addition of tubulin subunits. For net polymer elongation, the association of tubulin heterodimers 105 into the growing microtubule is faster than microtubule depolymerization. However, at steady state, growth of microtubule polymer due to αβ-heterodimer addition is counterbalanced by shrinkage due to disassembly into αβ-tubulin subunits. Thus, a polymerized microtubule 120 switches between episodes of growth and shrinkage, a property called dynamic instability (i.e., a normal equilibrium for the microtubule).

Taxanes 125 such as paclitaxel are microtubule-binding drugs that target specific sites within the lumen of polymerized microtubules 120. The taxanes 125 act by binding to GDP-bound β-tubulin molecules and stabilizing them by changing their conformation to a more stable GTP-bound β-tubulin structure. The taxanes cytotoxic effect is attributed to their ability to bind tubulin, stabilize the protofilaments leading to microtubule over-polymerization, and ultimately death by apoptosis. However, without being bound by theory, the reversible binding of taxanes 125 to the lumen of the polymerized microtubules 120 may hinder diffusion of the taxanes 125 along the microtubule axis, and thus affect tissue retention of the taxanes 125.

In order to overcome the aforementioned tissue retention and drug loss problems, various embodiments are directed to DCBs advantageously having a coating morphology that maintains the drug on a surface of the DCB during transport, inflation, and deflation of the DCB to a therapeutic location, e.g., an arterial wall, while also increasing tissue retention of the drug transferred from the DCB. For example, use of a removable cover on the DCB and/or a porous layer over a surface of the DCB are techniques that have been used to traditionally maintain the drug on a surface of the DCB during transport, inflation, and deflation of the DCB. However, such techniques alone or in combination have not been able to achieve optimal tissue retention of the drug while minimizing drug loss on a surface of the DCB during transport, inflation, and deflation of the DCB.

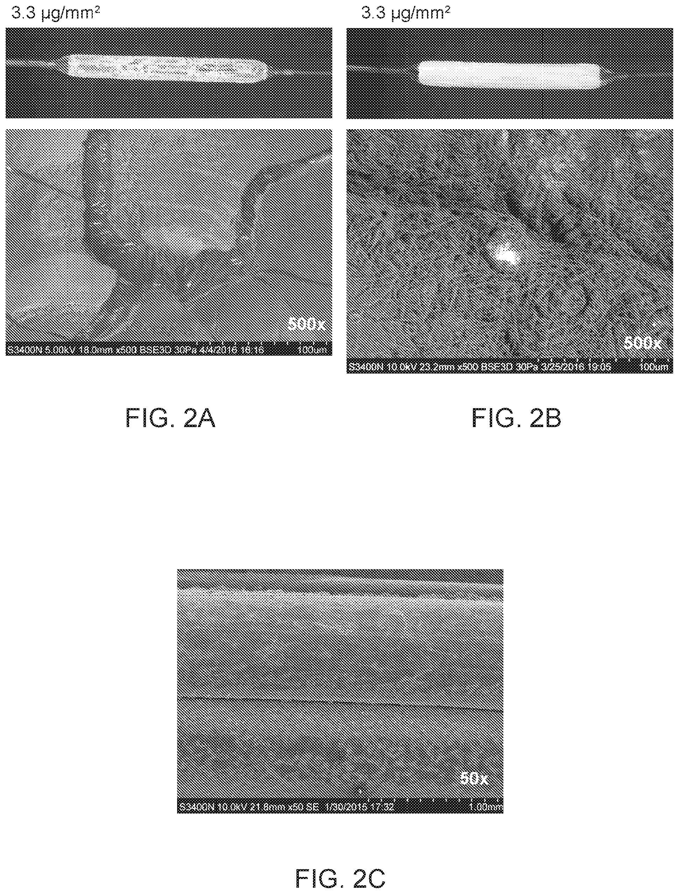

Nonetheless, it surprisingly has been discovered that the coating morphology of a DCB may be controlled independent of the drug dose to minimize drug loss during transport, inflation, and deflation while also maximizing tissue retention of the drug without the need of an additional cover/layer over the coating. As shown in FIGS. 2A, 2B, and 2C, imaging of the surfaces of various DCBs with similar drug doses (e.g., approximately 3.5 µg/mm² of a labeled amount of paclitaxel or 3.3 µg/mm² of a measured amount of paclitaxel) revealed different coating morphologies of the drug. For example, micrograph FIG. 2A at 500x magnification illustrates a comparative example of a DCB constructed of a Nylon balloon with a dose density of 3.5 µg/mm² and having a smooth coating morphology with cracks (e.g., caked or amorphous, fractured appearance), whereas micrograph FIG. 2B at 500x magnification and micrograph FIG. 2C at 50x magnification illustrate an example of a DCB constructed of a fluoropolymer with a dose density of 3.5 µg/mm² in accordance with various embodiments and having a microcrystalline surface coating morphology comprising microcrystals in a haystack orientation.

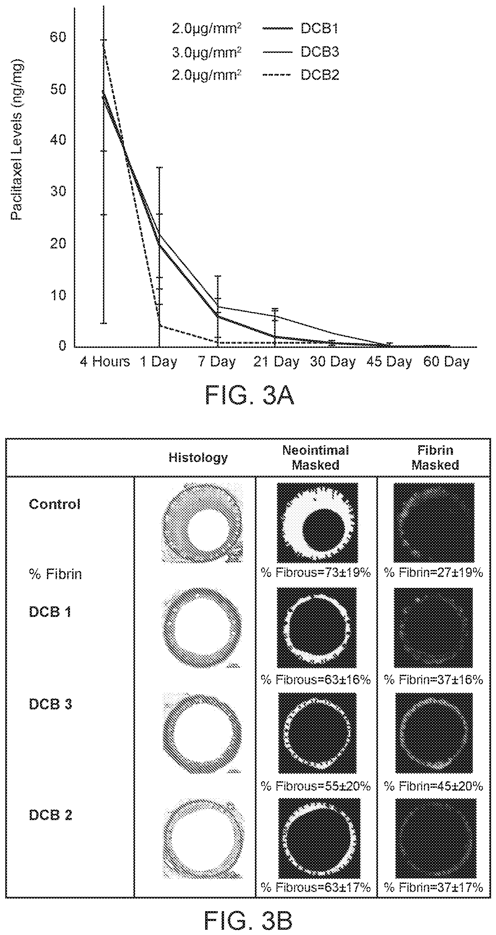

Additionally or alternatively, in order to overcome the aforementioned tissue retention problem, other embodiments are directed to techniques for increasing tissue retention of a drug transferred from a DCB. There are a number of techniques for increasing the tissue retention by modifying the dose of the drug on the balloon and/or preparing the vessel for absorption of the drug. For example, increasing the dose of the drug (e.g., FIG. 3A, which shows a comparison between DCB 1 having a dose density of 2.0 µg/mm² and DCB 2 having a dose density of 2.0 µg/mm² to DCB 3 having a dose density of 3.0 µg/mm², and the affect thereof on tissue retention) and/or preparing the vessel by using balloon angioplasty predilatation and/or atherectomy (e.g., laser, directional, rotational, orbital, or lithoplasty atherectomy) are techniques that have been used to traditionally increase tissue retention of a drug transferred from a DCB. However, such techniques alone or in combination have not been able to achieve optimal tissue retention of a drug delivered by a DCB. FIG. 3B shows the biological effects of the increase in dose density and tissue retention for DCB 1, DCB 2, and DCB 3.

Nonetheless, it surprisingly has now been discovered that utilization of DCBs having a fluoropolymer surface demonstrates that tissue retention from DCB benefits from repeat inflations, such as by inflating multiple balloons at the same treatment site, but not due to increased dose exposure, which has been hypothesized.. For example, although one skilled in the art would expect, at most, a 3x retention for 3x inflation using three DCBs, the results of repeated inflations or treatments provided a synergistic increase in tissue retention with greater than 3x retention, as demonstrated in the below Example 1. This demonstrates that repeated dilation (e.g., with a dose density of 3.6 to 4.1 µg/mm² on a DCB having a fluoropolymer surface with a porous microstructure) may prepare the blood vessel for enhanced absorption and/or overcome restrictions of single dose retention.

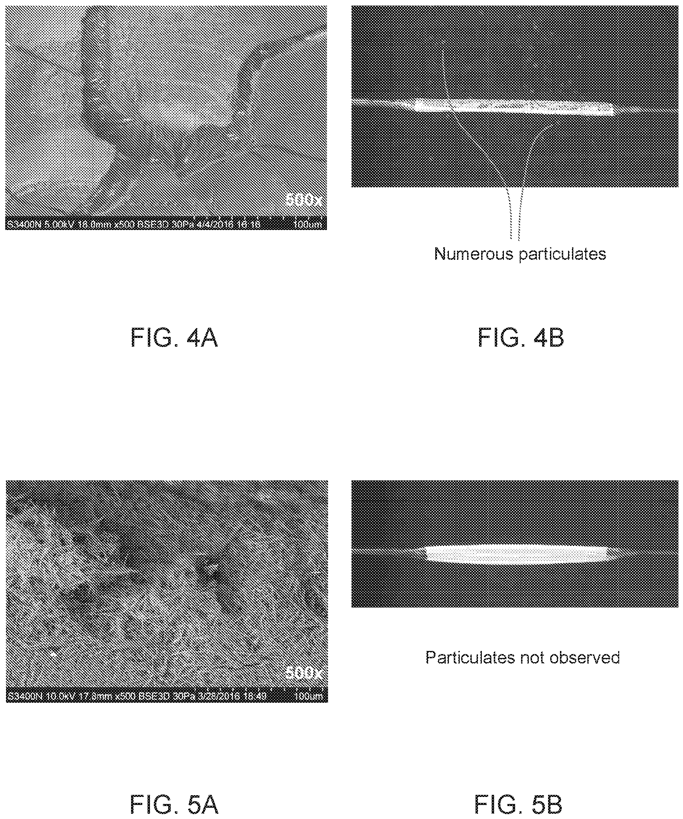

As further shown in FIGS. 4A, 4B, 5A, and 5B, imaging of various DCBs during inflation and deflation with similar drug doses (e.g., approximately 3.5 µg/mm² of paclitaxel) revealed differential particulation of the drug from the surface of the DCB. This may be dependent on the materials used to construct the various coatings and/or underlying balloon substrates, e.g., Nylon versus fluoropolymer. For example, FIGS. 4A and 4B illustrate a comparative example of a DCB constructed of a balloon with a Nylon surface/substrate having a drug dose density of about 3.5 µg/mm² and a smooth coating morphology with cracks (e.g., caked appearance or amorphous, fractured). As shown in FIG. 4B, the Nylon DCB exhibited particulation during inflation and deflation in a dry state. In contrast, FIGS. 5A and 5B illustrate an example of a DCB constructed of a balloon with a fluoropolymer surface/substrate having a dose density of about 3.5 µg/mm² and a microcrystalline surface coating morphology comprising microcrystals in a haystack orientation that was substantially devoid of particulation during inflation and deflation in a dry state in accordance with various embodiments.

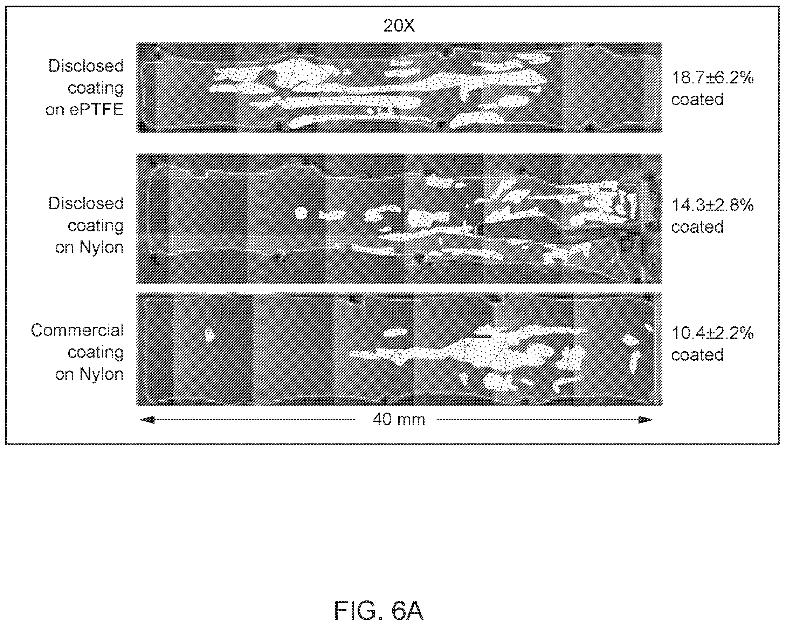

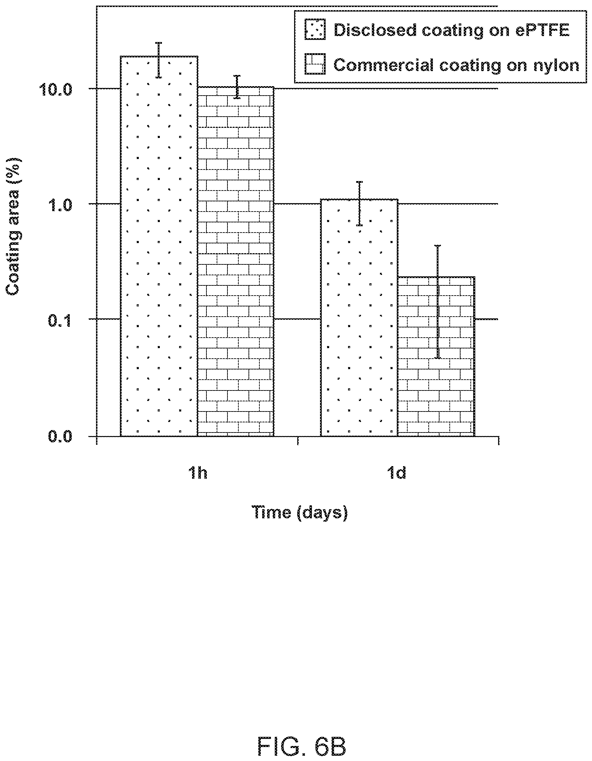

As further shown in FIGS. 6A and 6B, imaging of blood vessel surfaces treated using various DCBs revealed different coating formulations result in different drug delivery and drug retention efficiencies of the drug on the inner surface of the blood vessel that. For example, FIG. 6A is a micrograph at 20x magnification illustrating examples a therapeutic site treated with a commercial DCB as compared to DCBs according to aspects of the present disclosure. The micrographs shown in FIG. 6A depict three enface tissue coatings of a treatment site at 1 hour post treatment via three different DCBs, each inflated for 60 seconds. The first two DCBs were constructed in accordance with the present disclosure, one of expanded polytetrafluoroethylene (ePTFE), and one of nylon. The third was a commercial DCB constructed of Nylon, as described with respect to FIG. 2A. The enface tissue coating may be calculated based on Equation (1): Enface Tissue Coating = (Coated Area/Treated Area). The Coated Area corresponds to the area of the lumen or vessel along a treatment site that is visibly coated with the drug coating from the DCB. The "Treated Area" corresponds to the area of the treatment site. In the examples shown in FIG. 6A, the treatment site extended approximately 40 mm. A length of the treatment site may correspond to a working length of the DCB.

For the commercial Nylon DCB, FIG. 6A illustrates that the drug coating provided for a therapeutic site that exhibited about 10.4% (e.g., from 8% to 13%) enface tissue coating at 1 hour post treatment (as described with respect to FIG. 2A). The tissue delivery in the commercial example was provided via a DCB constructed of a Nylon balloon having a drug dose of 3.5 µg/mm² and a single treatment inflation time of 60 seconds. For the ePTFE DCB, in accordance with embodiments, FIG. 6A shows an example of tissue delivery provided for an entire therapeutic site that exhibited about 18.7 % enface tissue coating (e.g., from about 12% to about 25%) at 1 hour post treatment. The DCB in this aspect was constructed of a fluoropolymer (ePTFE) balloon (described in FIG. 2B) having a drug dose of 3.5 µg/mm² and a single treatment inflation time of 60 seconds. For the Nylon DCB, in accordance with embodiments, FIG. 6A shows an example of tissue delivery provided for an entire therapeutic site that exhibited about 14.3% enface tissue coating (e.g., from about 11% to about 17%) at 1 hour post treatment. The DCB in this aspect was constructed of a nylon balloon having a drug dose of 3.5 µg/mm² and a single treatment inflation time of 60 seconds.

FIG. 6B is a chart illustrating a commercial example of a Nylon DCB (as described in FIG. 2A) of tissue delivery provided for an entire therapeutic site that exhibited about 10% (e.g, from 8% to 13%) enface tissue coating at 1 hour post treatment and about 0.2% enface tissue coating at 1 day post treatment. FIG. 6B also shows an example of tissue delivery for a fluoropolymer (ePTFE) DCB, in accordance with various embodiments (described in FIG. 2B), provided for an entire therapeutic site that exhibited about 18% (e.g., from 12% to 25%) enface tissue coating at 1 hour post treatment and about 1.1% enface tissue coating at 1 day post treatment. FIG. 6B also shows an example of tissue delivery for a Nylon DCB, in accordance with various embodiments provided for an entire therapeutic site that exhibited about 14% (e.g., from 11% to 17%) enface tissue coating at 1 hour post treatment and about .6 % (e.g., from .3 to .9%) enface tissue coating at 1 day post treatment.

Without being bound by theory, it has been demonstrated that the microcrystalline surface coating morphology comprising microcrystals in a haystack orientation may determine distribution of the drug on the inner surface of the blood vessel and in some manner may maximize tissue retention of the drug. In addition, the microcrystalline surface coating morphology comprising microcrystals in a haystack orientation may minimize drug loss during transport and/or during inflation and deflation without the need of an additional cover/layer over the drug coating, as demonstrated in the below Example 2. Thus, it surprisingly has now been discovered that utilization of DCBs having microcrystalline surface coating morphology comprising microcrystals in a haystack orientation on a fluoropolymer surface minimized drug loss during transport, inflation, and deflation without the need of an additional cover/layer over the drug coating. Although one skilled in the art would expect that a DCB having an amorphous coating morphology, for example an amorphous, fractured appearance as shown in FIG. 2A, will also have less particulation over that of a DCB having a microcrystalline surface coating morphology, the results of DCBs having a fluoropolymer surface and a microcrystalline surface coating morphology comprising microcrystals in a haystack orientation provided a synergistic increase in retaining the drug on the surface of the balloon during transport, inflation, and deflation. These results demonstrate that a drug-coated balloon with a fluoropolymer surface having a porous microstructure may promote microcrystalline growth from the porous microstructure and/or overcome particulation of the drug coating during transport, inflation, and deflation.

Without being bound by theory, it has been demonstrated that the microcrystalline surface coating morphology comprising microcrystals in a haystack orientation may improve distribution of the drug on the inner surface of the blood vessel when positioned on either a porous or a non-porous polymer layer of a balloon.

Moreover, it surprisingly has now been discovered that utilization of drug-coated balloons with a fluoropolymer surface in our study demonstrated that tissue retention from DCB benefits from a microcrystalline surface coating morphology comprising microcrystals in a haystack orientation but not due to increased dose exposure, which has been hypothesized. Although one skilled in the art would expect that a DCB that delivers a higher initial dose exposure over that of another DCB will also have a higher retainable dose at 1 hour over that of the another DCB, the results of DCBs with a fluoropolymer surface and a microcrystalline surface coating morphology comprising microcrystals in a haystack orientation provided a synergistic increase in tissue retention, as shown in the below Example 2. This demonstrates that microcrystalline surface coating morphology on a drug-coated balloon with a fluoropolymer surface having a porous microstructure may prepare the artery for enhanced absorption and/or overcome restrictions of poor drug deliverance.

### II. Drug Coated Balloons (DCBs)

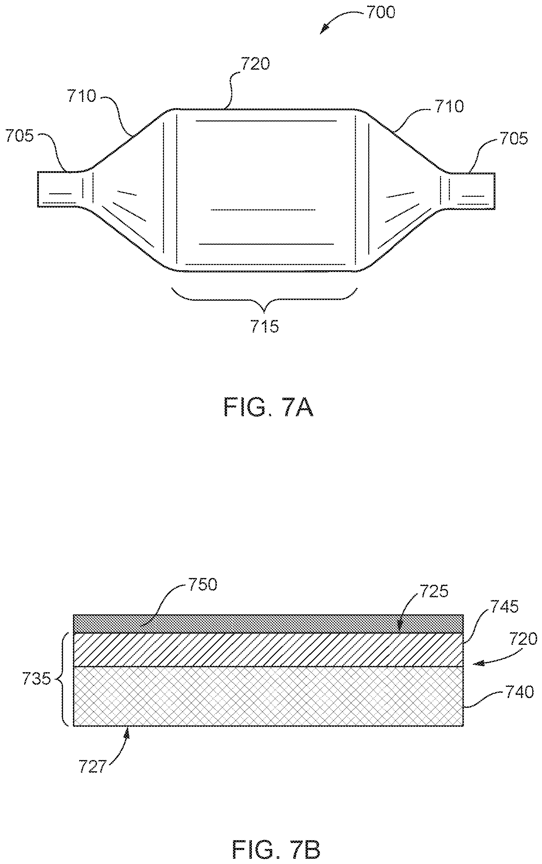

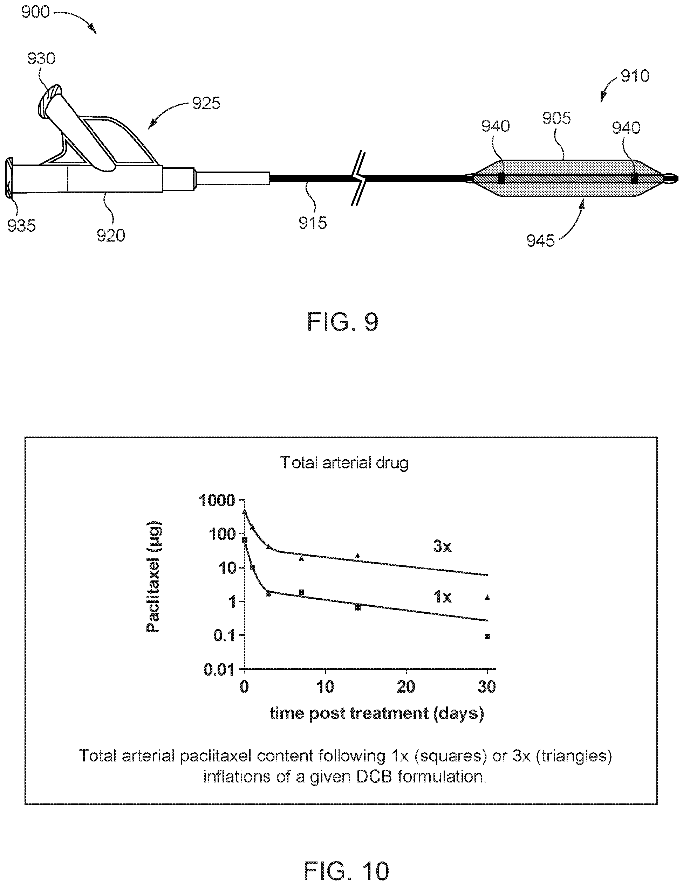

In various embodiments, a medical device is provided that comprises a balloon comprising an outer surface and a drug coating layer on the outer surface of the balloon. The drug coating comprises microcrystals in a haystack orientation applied evenly across the outer surface of the balloon. The balloon can have any appropriate dimension and size for the clinical application. In some embodiments, the balloon is substantially cylindrical along the working length. In some embodiments, the balloon may be a wrapped balloon, for example a balloon helically wrapped at an angle between 20 degrees and 90 degrees or 40 degrees and 70 degrees, or other suitable wrap angles. In some embodiments, the balloon may be a wrapped balloon that is wrapped at approximately a 90 degree angle (i.e., a cigarette wrapped balloon). As shown in FIG. 7A, balloon 700 has two opposed leg portions 705 that are integrally connected to shoulder/tapered portions 710. For the purposes of this disclosure, "working length" is defined as the length of the straight body section 715 of the balloon 700 which comprises the approximate length between the opposed shoulder/tapered portions 710. Leg portions 705, shoulder/tapered portions 710, and straight body section 715 define an "overall length" of the balloon 700. The working length of the balloon 700 can be about 10 mm to about 150 mm or more. Similarly, a nominal diameter of the balloon 700 can be about 2 mm to about 30 mm or more. By way of example, the balloon 700 can have a 4 mm diameter and a 30 mm working length, or alternatively, an 8 mm diameter and about a 60 mm working length. Of course, the balloon of the present disclosure can be constructed at any dimensions appropriate for the specific use. The balloon 700 may be attached or mounted to a catheter (as shown in FIG. 9) at the leg portions for delivery of a drug coating via inflation of the balloon 700 in the vasculature. The catheter may have one or more lumens, one of which may be in communication with the chamber of the balloon 100 for supplying inflation fluid to inflate the balloon 700.

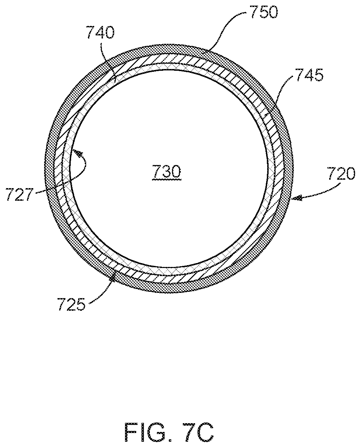

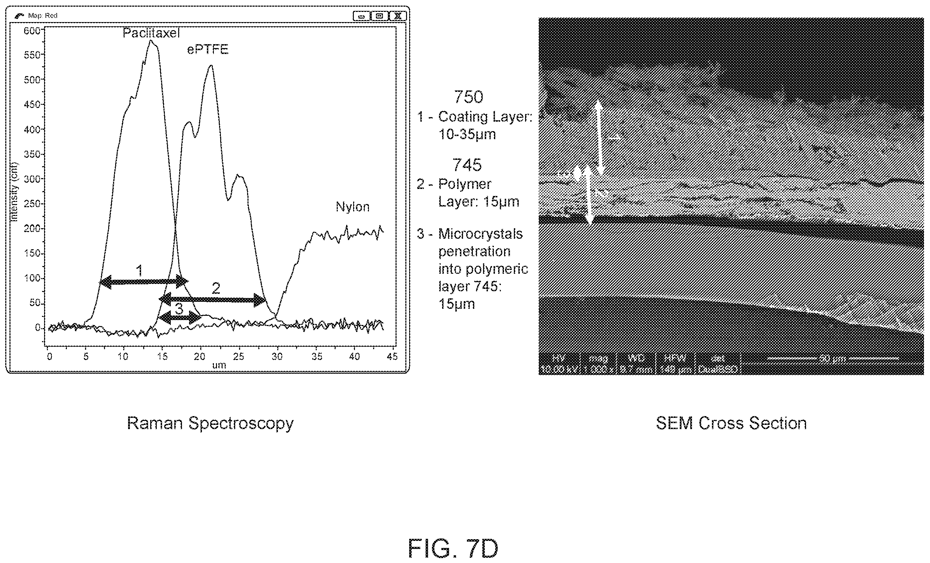

With reference to FIGS. 7A, 7B, 7C, and 7D, the balloon 700 may further comprise a balloon wall 720 comprising an outer surface 725 and an inner surface 727. The balloon wall 720 defines a chamber 730 and may be constructed of a layered material 735. In some embodiments, the layered material 735 comprises a thermoplastic polymeric layer 740 at least partially adhered to a substrate or polymeric layer 745 in an overlying relationship to each other. In certain embodiments, the layered material 735 can be created through a stretch blow molding process, as described in U.S. Patent Application Publication No. 2016/0106961. In other embodiments, the layered material 735 can be created by wrapping (e.g., a helical wrap) one layer around another layer, for example, the polymeric layer 745 may be wrapped around the thermoplastic polymeric layer 740, as described in U.S. Patent Application Publication No. 2016/0143759 A1. The layered material 735 may be constructed by blow mold or wrapping such that a thickness of the thermoplastic polymeric layer 740 is from 10 µm to 40 µm, for example, from 15 µm to 35 µm or about 30 µm, and a thickness of the polymeric layer 745 is from 5 µm to 40 µm, for example, from 10 µm to 30 µm or about 15 µm.

In some embodiments, the thermoplastic polymeric layer 740 defines the inner surface of the balloon wall 720, which serves as a bladder to retain the inflation fluid, and thus is composed of an impermeable or fluid-tight material. In accordance with such aspects, the polymeric layer 745 or other layer of material (e.g., a second polymeric layer) defines the outer surface 725 of the balloon wall 720. In alternative embodiments, the polymeric layer 745 defines the inner surface of the balloon wall 720, which serves as a bladder to retain the inflation fluid, and thus is composed of an impermeable or fluid-tight material. In accordance with such aspects, the thermoplastic polymeric layer 740 or other layer of material (e.g., a second polymeric layer) may define the outer surface 725 of the balloon wall 720.

In various embodiments, a coating layer 750 (e.g., a drug coating) is distributed evenly across at least a portion of the outer surface 725 of the layered material 735. For example, as shown in FIGS. 7B, 7C, and 7D, a coating layer 750 may be distributed evenly across at least a portion of the outer surface of the polymeric layer 745. In some embodiments, the coating layer 750 is distributed evenly across only substantially the working length of the balloon 700. In other embodiments, the coating layer 750 is distributed evenly across substantially the working length of the balloon 700 and at least a portion of the leg portions 705 and/or shoulder/tapered portions 710. An "even distribution," "distributed evenly," and the like, means that a thickness of the coating layer 750 across the portion of the outer surface 725 is maintained within a certain percentage of a specified thickness, where the percentage includes 3, 10, and 20 percent. In certain embodiments, a thickness of the coating layer 750 is from 5 µm to 50 µm, for example, from 10 µm to 35 µm.

As shown in FIG. 7D, the thickness of the layered material 735 and the coating layer 750 was studied by Raman spectroscopy and scanning electron microscopy techniques, and it was found that the coating layer 750 may have an average penetration depth from 2 to 10 µm (e.g., about 5 µm) of the polymeric layer 745 having a porous microstructure, and thus may infiltrate the outermost layer of a porous microstructure cover (e.g., expanded polytetrafluoroethylene (ePTFE)). In contrast, the coating layer 750 does not penetrate into a polymeric layer 745 having a non-porous microstructure, and thus would be fully disposed on the outermost layers (e.g., outer two layers) of a non-porous microstructure cover (e.g., Nylon). Accordingly, without being bound by theory, a polymeric layer 745 having a porous microstructure, such as ePTFE, provides a sponge-like scaffold for the coating layer 750 (e.g., paclitaxel/stearic acid/tris coating) with a coating penetration to a depth of about 5 µm.

In some embodiments, a thickness of the coating layer 750 and the polymeric layer 745 is from 5 µm to 45 µm, for example, from 10 µm to 35 µm or about 25 µm. In some embodiments, a thickness of the coating layer 750, the thermoplastic polymeric layer 740, and the polymeric layer 745 is from 30 µm to 60 µm, for example, from 40 µm to 55 µm or about 45 µm. A ratio of a thickness of the thermoplastic polymeric layer 740 to a thickness of the polymeric layer 745 may from 1.5:1 to 2.5:1, for example about 2:1 or about 30 µm of thermoplastic polymeric layer 740 to 15 µm of polymeric layer 745. A ratio of a thickness of the thermoplastic polymeric layer 740 to a thickness of (the polymeric layer 745 and the coating layer 750) may from 1:1 to 1.7:1, for example about 1.2:1 or about 30 µm of thermoplastic polymeric layer 740 to 25 µm of (the polymeric layer 745 and the coating layer 750).

The thermoplastic polymeric layer 740 may be comprised of a compliant, semi-compliant or non-compliant thermoplastic polymer. Suitable thermoplastic polymers include polymers that are medical grade and are blow moldable. Examples of suitable thermoplastic polymers can include polymethyl methacrylate (PMMA or Acrylic), polystyrene (PS), acrylonitrile butadiene styrene (ABS), polyvinyl chloride (PVC), modified polyethylene terephthalate glycol (PETG), cellulose acetate butyrate (CAB); semi-crystalline commodity plastics that include polyethylene (PE), high density polyethylene (HDPE), low density polyethylene (LDPE or LLDPE), polypropylene (PP), polymethylpentene (PMP); polycarbonate (PC), polyphenylene oxide (PPO), modified polyphenylene oxide (Mod PPO), polyphenylene ether (PPE), modified polyphenylene ether (Mod PPE), thermoplastic polyurethane (TPU); polyoxymethylene (POM or Acetal), polyethylene terephthalate (PET, Thermoplastic Polyester), polybutylene terephthalate (PBT, Thermoplastic Polyester), polyimide (PI, Imidized Plastic), polyamide-imide (PAI, Imidized Plastic), polybenzimidazole (PBI, Imidized Plastic); polysulfone (PSU), polyetherimide (PEI), polyether sulfone (PES), polyaryl sulfone (PAS); polyphenylene sulfide (PPS), polyether ether ketone (PEEK); fluoropolymers that include fluorinated ethylene propylene (FEP), ethylene chlorotrifluoroethylene (ECTFE), ethylene tetrafluoroethylene (ETFE), polychlorotrifluoroethylene (PCTFE), polyvinylidene fluoride (PVDF), perfluoroalkoxy (PFA), or combinations, copolymers, or derivatives thereof. Other commonly known medical grade materials include elastomeric organosilicon polymers, and polyether block amide (e.g., PEBAX®). In particular, polyamides may include Nylon 12, Nylon 11, Nylon 9, Nylon 6/9, and Nylon 6/6. In certain embodiments, PET, Nylon, and PE may be selected for medical balloons used in coronary angioplasty or other high pressure applications. The specific choice of materials may depend on the desired characteristics/intended application of the balloon.

The polymeric layer 745 may be comprised of a compliant, semi-compliant or non-compliant polymer. Suitable polymers include a porous microstructure or a non-porous microstructure. In embodiments having a porous microstructure (referred to herein as a "porous layer"), suitable polymers include fluoropolymers, including without limitation perfluoroelastomers and the like, polytetrafluoroethylene and the like as well as expanded fluoropolymers. In embodiments having a non-porous microstructure (referred to herein as a "non-porous layer"), suitable polymers include polyamides including without limitation Nylon 12, Nylon 11, Nylon 9, Nylon 6/9, and Nylon 6/6.

The architecture of the porous microstructure may be selected based on the needs of the intended application. In some embodiments, the porous microstructure may be substantially fibrillated (e.g., a non-woven web having a microstructure of substantially only fibrils, some fused at crossover points or with smaller nodal dimensions). In other embodiments, the porous microstructure can comprise large nodes or large densified regions that may have an impact on the extent of compressibility/collapsibility of the material during blow molding. In still other embodiments, the porous microstructure can be a node and fibril microstructure somewhere between the aforementioned embodiments. In some embodiments, the porous microstructure can have an "open" microstructure such that the outer layer can have more loft and/or a drug coating layer can have more void space to occupy near the surface of the outer layer. Other examples of porous architectures can be fibrous structures (such as woven or braided fabrics), non-woven mats of fibers, microfibers, or nanofibers, flash spun films, electrospun films, and other porous films.

In some embodiments, the porous microstructure may be comprised of expanded fluoropolymers or expanded polyethylene (see e.g., U.S. Patent No. 6,743,388 to Sridharan et al.). Non-limiting examples of expanded fluoropolymers include, but are not limited to, ePTFE , expanded modified PTFE, and expanded copolymers of PTFE. Patents have been filed on expandable blends of PTFE, expandable modified PTFE, and expanded copolymers of PTFE, such as, for example, U.S. Patent No. 5,708,044 to Branca; U.S. Patent No. 6,541,589 to Baillie; U.S. Patent No. 7,531,611 to Sabol et al.; U.S. Patent 8,637,144 to Ford; and U.S. Patent 8,937,105,to Xu et al.

The polymeric layer 745 may be formed from a tubular member of a polymer having the porous or nonporous microstructure. The tubular member can be formed as an extruded tube or can be film-wrapped. The tubular member can have circumferential, helical, or axial orientations of the microstructure. In various embodiments, the tubular member can be formed by wrapping a film or tape and the orientation can be controlled by the angle of the wrapping. Tubular member can be circumferentially wrapped or helically wrapped. When a porous material is wrapped helically versus circumferentially or axially, the degree of compliancy in a given direction can be varied and can influence the overall compliancy of the composite. (As used herein, the term "axial" is interchangeable with the term "longitudinal." As used herein, "circumferential" means an angle that is substantially perpendicular to the longitudinal axis.)

The coating layer 750 may be comprised of at least one natural, semi-synthetic or synthetic therapeutic agent (e.g., at least one drug). The functional characteristic of the coating layer 750 is to allow for release of at least one therapeutic agent to the tissue of a vascular wall during balloon inflation (e.g., treatment via percutaneous transluminal angioplasty in patients with obstructive disease of the peripheral arteries). In certain embodiments, the therapeutic agent is either lipophilic (partition coefficient between n-butanol and water >10) or displays very poor water solubility (<10 mg/ml, 20°C). The wording "at least one therapeutic agent (or therapeutic agent preparation)" means that a single therapeutic agent or mixtures of different therapeutic agents are included. Thus, various therapeutic agents may be applied or combined, if different pharmacological actions are required or efficacy or tolerance is to be improved.

Therapeutic agents suitable for use in the coating layer 750 may include inhibitors of restenosis or cell proliferation (e.g., an anti-mitotic drug or anti-proliferative drug) such as vinco alkaloids, e.g., colchicine, podophyllotoxin, griseofulvin, anlimitotic alkaloid agents, and anti-microtubule alkaloid agents, and taxanes, e.g., paclitaxel, docetaxel, and protaxel. In certain embodiments, the therapeutic agent comprises paclitaxel or arsenic trioxide. Alternatively, therapeutic agents suitable for use in the coating layer 750 may include specific inhibitors of neovascularization such as thalidomide, statins like atorvastatin, cerivastatin, fluvastatin or antiinflammatory drugs like corticoids or even lipophilic derivatives of corticoids such as betamethasone diproprionate or dexamethasone21-palmitate, and Limus drugs, especially immuno-suppressants and mitosis inhibitors like mTOR inhibitors such as sirolimus, everolimus, zotarolimus, biolimus, temsirolimus. That is, for some embodiments the therapeutic agent comprises paclitaxel, a taxane, docetaxel, vinca alkaloids, colchicine, podophyllotoxin, griseofulvin, anti-mitotic alkaloid agents anti-microtubule alkaloid agents, protaxel, arsenic trioxide, thalidomide, atorvastatin, cerivastatin, fluvastatin, betamethasone diproprionate, dexamethasone 21-palmitate, sirolimus, everolimus, zotarolimus, biolimus or temsirolimus. As should be understood the at least one therapeutic agent may include structural analogs, related substances, degradants and derivatives of any of the afore-mentioned drugs.

The coating layer 750 may be further comprised of an excipient including one or more saturated or unsaturated fatty acids. The excipient is a substance formulated alongside the therapeutic agent, and included for the purpose of long-term stabilization, bulking up solid formulations that contain potent active ingredients (thus often referred to as "bulking agents," "fillers," or "diluents"), or to confer a therapeutic enhancement on the therapeutic agent in the final dosage form, such as facilitating drug absorption, reducing viscosity, or enhancing solubility. Suitable excipients for use include one or more monocarboxylic acid salts (chain length C₆-C₃₀), preferably with Mg²⁺, Ca²⁺, Zn²⁺ or ammonium (NH⁴⁺), more preferably with Mg²⁺, Ca²⁺ or Zn²⁺. The C₆-C₃₀-monocarboxylic acid may be saturated or may have one or more double bonds (unsaturated). Examples of C₆-C₃₀-monocarboxylic acid salts are magnesium stearate, calcium stearate, zinc stearate, magnesium palmitate, calcium palmitate, zinc palmitate, magnesium myristate, calcium myristate, magnesium laurate, calcium laurate, magnesium caprinate, calcium caprinate, magnesium caprylate, calcium caprylate, magne-sium oleate, calcium oleate, magnesium palmitoleate or calcium palmitoleate. Optionally, the salts are admixed to at least one of stearic acid, palmitic acid, lauric acid, capric acid, caprylic acid, oleic acid, palmitoleic acid, stearic alcohol, palmitic alcohol, lauric alcohol, magnesium acetate and/or calcium acetate. That is, for some embodiments the C₆-C₃₀-monocarboxylic acid salt comprises a magnesium salt, calcium salt, zinc salt, or ammonium salt. In other embodiments, the C₆-C₃₀-monocarboxylic acid salt comprises magnesium stearate, calcium stearate, zinc stea-rate, magnesium palmitate, calcium palmitate, zinc palmitate, magnesium myristate, calcium myristate, magnesium laurate, calcium laurate, magnesium caprinate, calcium caprinate, magnesium caprylate, calcium caprylate, magne-sium oleate, calcium oleate, magnesium palmitoleate, calcium palmitoleate, tris stearate, lysine stearate, or arginine stearate. In yet other embodiments, the C₆-C₃₀-monocarboxylic acid salt comprises tris stearate, lysine stearate, or arginine stearate

In some embodiments, the C₆-C₃₀-monocarboxylic acid salt is a derivative of a C₆-C₃₀-monocarboxylic acid and a base. That is, for certain embodiments the C₆-C₃₀-monocarboxylic acid comprises stearic acid, palmitic acid, lauric acid, arachidic acid, capric acid, caprylic acid, myristic acid, behenic acid, lignoceric acid, cerotic acid, oleic acid, palmitoleic acid, Sapienic acid, elaidic acid, vaccenic acid, linoleic acid, linoelaidic acid, α-Linolenic acid, Arachidonic acid, Eicosapentaenoic acid, stearic alcohol, palmitic alcohol, caprylic alcohol, capric alcohol, lauric alcohol, myristic alcohol, arachidic alcohol, behenic alcohol, lignoceric alcohol, cerotic alcohol, magnesium acetate, or calcium acetate. The base may be an organic base such as a monovalent amine selected from the group consisting of ethanolamine, aminopropandiole, serinol, glucosamine, tris(hydroxymethyl)aminomethane, methylglu-camine, and an amino acid. In yet other embodiments, the salt may comprise C₆-C₃₀-monocarboxylic acid, tris stearate and reaction products of tris stearate with ethylene oxide.

The coating layer 750 may comprise the therapeutic agent and the excipient in a predetermined weight ratio calculated based on a formulation solution of the coating layer 750. In certain embodiments, the predetermined weight ratio (drug : excipient) is greater than 2:1, for example in terms of ranges between 3:1 and 20:1, and preferably greater than 3:1, for example in terms of ranges between 4:1 and 19:1, or about 19:1. A dose density of the therapeutic agent (e.g., 3.5 µg/mm²) is selected to provide a clinical dose of the therapeutic. The dose density of the therapeutic argent is the mass of the therapeutic agent within the portion of the outer surface 725 of the layered material 735. In some embodiments, the dose density of the therapeutic agent is from 2.0 to 7.0 µg/mm², e.g., 3.3 to 3.8 µg/mm² or from 3.6 to 4.1 µg/mm². In other embodiments, the dose density is selected to provide a percentage (e.g., less than 100%) of the clinical dose of the therapeutic agent, for example a half (e.g., 1.8 to 2.05 µg/mm²), a third (e.g., 1.2 to 1.4 µg/mm²), a fourth (e.g., 0.9 to 1.03 µg/mm²) of the clinical dose. A dose density of the excipient is selected such that the predetermined weight ratio is maintained within the coating layer 750. The dose density of the excipient is the mass of the excipient within the portion of the outer surface 725 of the layered material 735. In some embodiments, the dose density of the total excipient mass is from 0.1 to 0.7 µg/mm², e.g., 0.1 to 0.5 µg/mm² or from 0.1 to 0.3 µg/mm². In other embodiments, the dose density is expressed in terms of its individual constituents, where the dose density of the C₆-C₃₀-monocarboxylic acid is from 0.05 to 0.3 µg/mm², e.g., 0.08 to 0.15 µg/mm² and the dose density of the base is from 0.02 to 0.1 µg/mm², e.g., 0.03 to 0.08 µg/mm².

Typically, the therapeutic agent and mixtures of therapeutic agents with additives such as excipients are coated on medical devices (e.g., a balloon) as liquid formulations in volatile solvents. In various embodiments, the formulation for the coating layer 750 comprises at least one therapeutic agent, an excipient, and a solvent. The choice of the solvent may be useful for the crystal morphology of the coating layer 750 in a dry state and adherence and release of the therapeutic agent from the surface of the medical device. Accordingly, the solvent may include acetone, dioxane, tetrahydrofuran (THF), water, and mixtures thereof.

The choice of solvent must also consider toxicity and biocompatibility. The choice of the solvent, in accordance with various aspects discussed herein, was modified from conventional solvents formulations to remove a polar solvent, such as tetrahydrofuran (THF), in favor of components to enhance drug release of the therapeutic agent from the surface of the medical device, e.g., acetone and water. The polar solvent was initially included in the conventional solvent formulations to prevent precipitation of the therapeutic agent (e.g., about 25 mg/ml) in the coating solution but it did not enhance drug release. It has been found that dioxane may be substituted for the polar solvent because of its lower toxicity and higher boiling point; however, it was also found that the water content of the solvent formulation should be increased, resulting in a solvent system of acetone, dioxane, and water (e.g., 58:14:28 % by volume respectively). Accordingly, the solvent may include acetone, dioxane, water, and mixtures thereof and optionally include acetone, water, and mixtures thereof.

The solvent dioxane provides acceptable drug release and transfer; however, while dioxane is less toxic than some conventional solvents, safety concerns still persist when handling and storing this solvent. The solvent formulation was further modified to remove dioxane; however, it was found that the therapeutic agent concentration should be lowered (e.g., about 17.5 mg/ml) resulting in a solvent system of acetone and water (e.g., 75:25 % by volume respectively). Accordingly, in some embodiments, the solvent comprises acetone, dioxane, and water. In other embodiments, the solvent consists essentially of acetone, dioxane, and water. In alternative embodiments, the solvent comprises acetone and water. In yet other embodiments, the solvent consists essentially of acetone and water. In some embodiments, the solvent comprises from 35% to 85% by vol. of acetone, for example, about 75% or 0.75 v/v, and from 5% to 35% by vol. of water, for example, about 25% or 0.25 v/v. In other embodiments, the solvent comprises from 35% to 65% by vol. of acetone, for example, about 58% or 0.58 v/v, from 5% to 35% by vol. of water, for example, about 28% or 0.27 v/v, and from 5% to 30% by vol. of dioxane, for example, about 14% or 0.14 v/v.

In various embodiments, the formulation for the coating layer 750 comprises from 10 mg/ml to 30 mg/ml of at least one therapeutic agent, for example about 17.5 mg/ml; from 0.5 mg/ml to 3.5 mg/ml of excipient (in certain embodiments, from 0.2 mg/ml to 1.5 mg/ml of a C₆-C₃₀-monocarboxylic acid, for example, 0.62 mg/ml, and from 0.05 mg/ml to 0.7 mg/ml of a base, for example, 0.26 mg/ml); from 35% to 85% by vol. of acetone, for example, about 75% or 0.75 v/v; and from 5% to 35% by vol. of water, for example, about 25% or 0.25 v/v. In other embodiments, the formulation for the coating layer 750 comprises from 15 mg/ml to 35 mg/ml of at least one therapeutic agent, for example, about 25.0 mg/ml; from 0.5 mg/ml to 4.0 mg/ml of excipient (in certain embodiments, from 0.2 mg/ml to 1.8 mg/ml of a C₆-C₃₀-monocarboxylic acid, for example, about 0.88 mg/ml, and from 0.05 mg/ml to 0.9 mg/ml of a base, for example, about 0.37 mg/ml); from 35% to 65% by vol. of acetone, for example, about 58% or 0.58 v/v; from 5% to 35% by vol. of water, for example, about 28% or 0.27 v/v; and from 5% to 30% by vol. of dioxane, for example, about 14% or 0.14 v/v. However, one skilled in the art will understand that modifications of this formulation may be acceptable provided that the modifications do not change the primary constituents and may include, without limitation, modifications that involve similar derivatives and analogs to these primary constituents and/or involve additives to the formulation and/or its primary constituents.

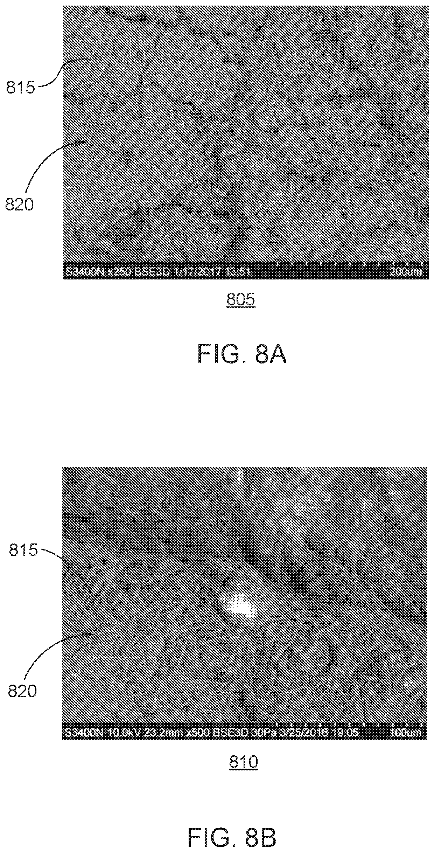

As discussed herein, various embodiments are directed to DCBs advantageously having a coating morphology that maintains the drug on a surface of the DCB during transport, inflation, and deflation of the DCB to a therapeutic location, e.g., an arterial wall, while also increasing tissue retention of the drug transferred from the DCB. As shown in FIGS. 8A and 8B, the coating layer formulation, established in accordance with the various embodiments described herein, provides a substantially similar microcrystalline morphology on non-porous (e.g., Nylon) 805 and porous balloon substrates (e.g. ePTFE) 810. The microcrystalline morphology may comprise microcrystals 815 in a haystack orientation 820. For example, the microcrystals 815 may be uniformly distributed across the substrate, and demonstrate a random and a substantial absence of uniformity (non-uniform) in placement on the substrate and/or random and a substantial absence of uniformity (non-uniform) in angle of projection from the substrate. The terms "angle," "projection angle," "angle of projection," and the like, are the geometric angle that a projecting object has relative to the outermost plane of the substrate surface. "Placement" and the like, means a rotation or offset that an object has relative to a central axis of the outermost plane of the substrate surface. A "uniform distribution," "uniformly distributed," and the like, means that a percentage by volume of the microcrystals 815 is maintained within a certain percentage, where the certain percentage includes 3, 10, and 20 percent. In some embodiments, a percentage by volume of the microcrystals 815 is from 50% to 100%, for example, from 65% to 85%.

According to the claimed invention, a majority of the microcrystals 815 extends from a surface of the substrate at an angle of less than 20° (thus the crystals lay relatively flat on the substrate). In other embodiments, a majority of the microcrystals 815 extend from the surface of the substrate at an angle of 0° to 17°, 5° to 15°, less than 15°, less than 10°, or less than 8°. Additionally, a majority of the microcrystals 815 each have a major dimension length that is at least ten (5) times greater than the major dimension width of the microcrystal. In other embodiments, a majority of the microcrystals 815 each have a major dimension length that is at least ten (10) times greater than the major dimension width of the microcrystal In other embodiments, a majority of the microcrystals 815 each have a major dimension length that is at least 13 or at least 15 times a major dimension width. Additionally, a majority of the microcrystals 815 optionally each have a major dimension length that is between 12 µm and 22 µm, for example between 14 µm and 20 µm or about 17 µm, and a majority of the microcrystals 815 each have a major dimension width that is between 0.5 µm and 2.0 µm, for example between 0.8 µm and 1.6 µm or about 1.3 µm.

As shown in FIG. 7D, the coating layer 750, and thus microcrystals (e.g., microcrystals 815), may penetrate into the outer 2-7 µm (e.g., about 5 µm) of a polymeric layer 745 having a porous microstructure, and thus may infiltrate the outermost layers (e.g., outer two layers) of a porous microstructure cover (e.g., expanded polytetrafluoroethylene (ePTFE)). In contrast, the coating layer 750, and thus microcrystals 815, do not penetrate into a polymeric layer 745 having a non-porous microstructure, and thus would be disposed substantially on the outermost layers (e.g., outer two layers) of a non-porous microstructure cover (e.g., Nylon). Accordingly, a polymeric layer 745 having a porous microstructure, such as ePTFE, provides a sponge-like scaffold for the coating layer 750 (e.g., paclitaxel/stearic acid/tris coating) with a coating penetration to a depth of about 5 µm, which may assist in minimizing the loss of drug during tracking and deployment as well as provide for better application.

Advantageously, the coating formulation and morphology allows for the therapeutic agent to adhere firmly enough to the substrate (porous and non-porous) to tolerate mechanical stress during production including folding of balloons, packaging, shipping to customers, and during final clinical use, which involves passage through a narrow hemostatic valve, an introductory sheath or guiding catheter, and a variable distance of possibly tortuous and narrow blood vessels. Moreover, the coating morphology is economical and efficient in manufacture as it does not require added costs or manufacturing steps to provide: a roughened balloon surface to enhance adherence, protective sheaths or membranes, or other physical or chemical methods to enhance adherence of the therapeutic agent to the balloon surface.

As shown in FIG. 9, a balloon catheter assembly 900 may comprise a balloon 905, for example the balloon 700 as described with respect to FIGS. 7A, 7B, 7C, and 7D, mounted on a distal section 910 of a catheter 915. The balloon catheter assembly 900 may also include a hub assembly 920 positioned on a proximal section 925 of the catheter 915. The hub assembly 920 may include an inflation port 930 that is in fluid communication with an inflation lumen of the catheter 915. The inflation lumen of the catheter 915 may be in fluid communication with an inner region of the balloon 905 such that an inflation medium may be inserted into the inflation port 930 to inflate the balloon 905. The hub assembly 920 may also include a second port 935 that is in communication with a central lumen of the catheter 915. The central lumen of the catheter 915 may extend from the proximal section 925 of the catheter 915 to the distal section 910 of the catheter 915 and may receive a guidewire. In some aspects the central lumen of the catheter 915 may also be used for flushing an inflation medium from the balloon 905. One or more radiopaque markers 940, for example but not limited to radiopaque platinum-iridium markers, may be positioned on the balloon 905 to indicate a working length of the balloon 905 and facilitate fluoroscopic visualization of the balloon 905 during delivery and placement. In some embodiments, the radiopaque marker may be positioned on the catheter 915 to indicate a working length of the balloon 905. As described above with respect to FIGS. 7B, 7C, and 7D, the balloon 905 includes a coating layer 945 on at least a portion of an outer surface of the balloon 905.

### III. Methods of Treatment Using DCBs

In accordance with various embodiments, a DCB (e.g., as a part of a balloon catheter assembly) may be utilized to provide one or more treatments at a desired site in a body lumen. The DCB may include a balloon having a coating layer on an outer surface of the balloon, as described with respect to FIGS. 7A, 7B, 7C, and 7D. Techniques for performing the one or more treatments may include positioning the DCB on a distal section of a balloon catheter assembly, as described with respect to FIG. 9, and advancing the DCB within a body lumen to a desired site. The location of the DCB or catheter may be monitored or tracked as it is advanced in the body lumen using radiopaque elements positioned on the DCB or catheter. Once the DCB has advanced to the desired site, the DCB may provide a single treatment (or inflation), or in other embodiments, may provide multiple or repeated treatments (or inflations) at the desired site. In various embodiments where the DCB provides a single treatment at the desired site, the DCB may be inflated for from 20 to 200 seconds, e.g., approximately 45 seconds, approximately 60 seconds, approximately 90 seconds, approximately 180 seconds or other suitable lengths of time. In other embodiments where the DCB provides a multiple or repeated treatments at the desired site, the DCB may be inflated for from 20 to 200 seconds, e.g., approximately 45 seconds, approximately 60 seconds, approximately 90 seconds, approximately 180 seconds or other suitable lengths of time for each treatment (or inflation). Following the one or more treatments, the DCB may be deflated and withdrawn from the body lumen.

As should be understood, the multiple or repeat treatments may be performed by inflating a single DCB multiple times at the same treatment site (as described in the above process), or by inflating multiple DCBs at a same treatment site multiple times (repeatedly performing the above process a number of times). For example, other aspects of the disclosure are directed to techniques of using the described DCBs in a sequential medical procedure. Such techniques can comprise passing the balloon catheter device with a DCB mounted thereon through an anatomical conduit or vessel to the desired position and inflating the described DCB to a nominal diameter once or sequentially. The method can further comprise expanding the balloon and delivering, upon inflation, a therapeutic agent that is on the outer surface of the balloon to a surrounding tissue or endovascular device. The method can further comprise sequentially retracting the balloon catheter device from the anatomical conduit or vessel and passing another DCB mounted to the balloon catheter device through the anatomical conduit or vessel to the desired position and inflating the subsequent balloon to a nominal diameter once or sequentially.

In accordance with various embodiments, the dose densities used for a single treatment utilizing a balloon comprising a fluoropolymer surface may provide for the release of from 68% to 83% of a loaded dose of the drug, e.g. between 72% and 77% of the loaded dose of the drug from the drug coating upon an inflation time of about 60 seconds. A loaded dose as used herein refers to the total mass of the drug on the balloon. In accordance with various embodiments, the dose densities used for a single treatment utilizing a 6mm x 40mm balloon comprising a fluoropolymer surface may provide for the release of from 2200.0 µg to 2700.0 µg, e.g., 2350.0 to 2500.0 µg of the drug from the drug coating upon an inflation time of about 60 seconds. The tissue delivery of the released drug at one hour after a single treatment exhibited from about 10.0% to 30.0% enface tissue coating, e.g., from about 14.0% to 21.0% enface tissue coating, and at one day after a single treatment exhibited from about 0.1% to 10.0% enface tissue coating, e.g., from about 0.7% to 4.0% enface tissue coating. The released drug and resulting enface tissue coating may provide an initial delivered dose of drug to the tissue of from 29% to 73% of a loaded dose of the drug, e.g., 44% to 67%. The loaded dose of the drug corresponds to the total amount of the drug on the balloon and may be determined based on the dose density of the drug and the size of the balloon. In some embodiments utilizing a 5mm x 40mm balloon, the initial delivery dose of drug to the tissue may be from about 750 to 1900 µg, e.g., 1150 µg to 1750 µg. Nonetheless, the dose amount retained by the tissue at one hour after the single treatment was surprisingly multiple times (e.g., greater than two times or greater than three times) the dose amount retained in the tissue at one hour after a single treatment of a traditional balloon. In some embodiments, the dose amount retained by the tissue at one hour after the single treatments is greater than two times, at least three times, at least four times, or at least five times the dose amount retained in the tissue at one hour after a single treatment of a traditional balloon. The dose amount retained in the tissue at one hour after a single treatment with a 5 x 40 mm balloon, for example, may be from 50 µg/g to 2000 µg/g. The dose amount retained in the tissue at one day after a single treatment with a 5mm x 40mm balloon, for example, may be from 1 µg/g to 1000 µg/g. In some embodiments, the dose amount retained in the tissue at one hour after a single treatment with a 6mm x 40mm balloon, for example, may be from 50.0 µg/g to 2000.0 µg/g. The dose amount retained in the tissue at one day after a single treatment with a 6mm x 40 mm balloon, for example, may be from 1 µg/g to 1000 µg/g.

In accordance with other embodiments, repeated treatments may be performed. In some embodiments, the dose densities used for each treatment may be the same or different dose densities from each other. Nonetheless, the dose amount retained by the tissue at one hour after the repeated treatments utilizing a balloon comprising a fluoropolymer surface was surprisingly multiple times (e.g., greater than three times) the dose amount retained in the tissue at one hour after a single treatment. In some embodiments, the dose amount retained by the tissue at one hour after the repeat treatments is greater than three times, at least four times, at least five times, at least six times, or at least seven times the dose amount retained in the tissue at one hour after a single treatment. The dose amount retained in the tissue at one hour after a single treatment with a 5 x 40 mm balloon may be from about 1% to about 10%, for example from 1% to 3% of the loaded dose of the drug on the balloon. In some embodiments, the dose amount retained in the tissue one hour after a single treatment with a 5 x 40 mm balloon may be from 60 to 90 µg/g, for example from 69.5 to 74.7 µg/g or from 71.5 to 72.7 µg/g. Surprisingly, however, the dose amount retained in the tissue at one hour after sequentially treating a vascular treatment site n times (e.g., three times) with the one or more medical devices (optionally using a same dose density for each treatment) may be greater than n times (e.g., three times) the dose amount retained in the tissue at one hour after a single treatment, for example, from 240 to 360 µg/g (four times), 300 to 450 µg/g (five times), 360 to 540 µg/g (six times), or 420 to 630 µg/g (seven times); from 278 to 298.8 µg/g (four times), 347.5 to 373.5 µg/g (five times), 417.0 to 448.2 µg/g (six times), or 486.5 to 522.9 µg/g (seven times); or from 286.0 to 290.8 µg/g (four times), 357.5 to 363.5 µg/g (five times), 429.0 to 436.2 µg/g (six times), or 500.5 to 508.9 µg/g (seven times).

Advantageously, the dose amount retained by the tissue after the repeated treatments was also surprisingly cleared at a slower rate. In some embodiments, a half-life of the drug retained by the tissue after the repeated treatments was greater than the half-life of the drug retained by the tissue after a single treatment. The half-life of the drug retained in the tissue after a single treatment may be from 6 to 8 hours. Surprisingly, however, the half-life of the drug retained in the tissue after repeated treatments (a plurality of treatments) (optionally using a same dose density for each treatment) may be greater than 8 hours, greater than 9 hours, greater than 10 hours, greater than 11 hours, greater than 12 hours, greater than 13 hours, greater than 14 hours, or greater than 15 hours. In terms of ranges, the half-life of the drug retained in the tissue after repeated treatments (optionally using a same dose density for each treatment) may be from 8 to 15, e.g., 10 to 14 hours.

### IV. Testing Methods

It should be understood that although certain methods and equipment are described below, any method or equipment determined suitable by one of ordinary skill in the art may be alternatively utilized.

### Mass, Thickness, and Mass per Unit Area

Membrane samples were die cut to form rectangular sections about 2.54 cm by about 15.24 cm to measure the weight (using a Mettler-Toledo analytical balance model AG204) and thickness (using a snap gauge-Mutitoyo Model, 547-400, 0.5" diameter foot). Using these data points, mass per unit area was calculated with the following formula: m/(w^{∗}l), in which: mass per unit area (g/cm²), m=mass (g), w=width (cm), and l=length (cm). The average of three measurements was reported.

### Bubble Point Test

The bubble point test is used for pore size determination. It is based on the fact that, for a given fluid and pore size with a constant wetting, the pressure required to force an air bubble through the pore is inverse proportion to the size of the hole. The given fluid used for the examples disclosed herein was isopropyl alcohol, and the isopropyl alcohol bubble point was measured in the following manner: The material was restrained with a circular fixture of 1 inch diameter. The material was subjected to pressurized air at a pressurization rate of about 0.2 psi/sec. The pressure was increased until a stream of bubbles appeared, followed by additional streams of bubbles at similar pressures. The reported values represent the average measurements for five samples.

### V. Examples

Without intending to limit the scope of the embodiments discussed herein, the systems and methods implemented in various embodiments may be better understood by referring to the following examples.

EXAMPLE 1: The preclinical performance of an embodiment of a DCB was evaluated where paclitaxel (PTX) was applied to an ePTFE porous surface of the balloon.

Methods: PTX coated balloons (W.L. Gore, Flagstaff AZ) with dose densities of 4.1 µg/mm² (5x40 mm) or 3.6 µg/mm² (6x40 mm) were inflated for 60 seconds in peripheral arteries of 30 Yorkshire swine (target balloon-to-artery ratio between 1.05:1-1.2:1). Arteries received a clinical dose via a single treatment or a safety margin dose via 3 sequential treatments with separate DCBs at the same angiographic site. Animals were euthanized from 1h to 30 days (n=4-8/time point) post-treatment and subjected to comprehensive necropsies. Treated arteries, along with other tissues, were collected for bioanalysis or processed for histologic and scanning electron microscopy (SEM) evaluation. The time series of total arterial drug for each treatment group was fit to a bi-exponential model with zero plateau (R2≥0.999) and fits compared using a paired t-test.

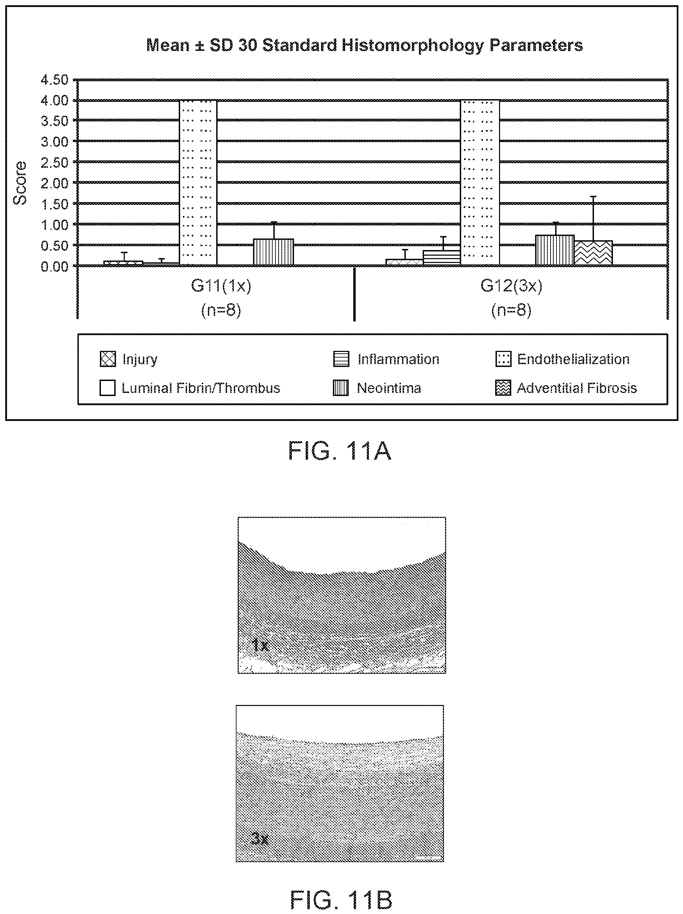

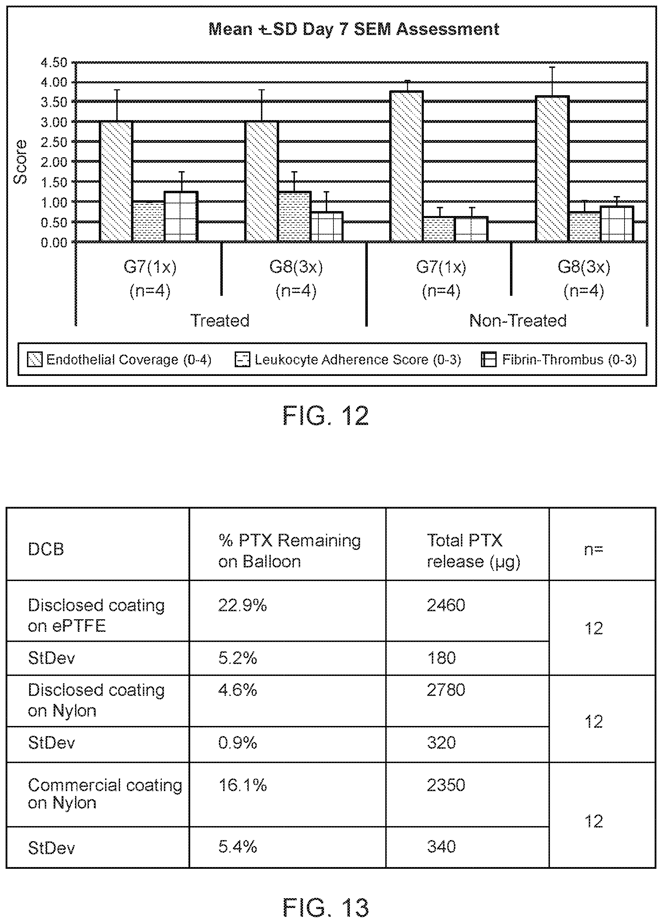

Results: DCB treatments were successful in peripheral vessels, with absence of adherent thrombus on removed catheters and survival of all animals to scheduled time points. Devices released 74.6% ± 4.6% of PTX load during treatment. As shown in FIG. 10, at clinical dosage (1x), maximum PTX concentration in arteries was 806 ± 676 ng/mg at 1 hour, decreasing to 0.22 ± 0.20 ng/mg by 30 days. At 3x safety margin dose, maximum PTX concentration in arteries was almost 7-fold higher at 5318 ± 5110 ng/mg at 1 hour, decreasing to 3.06 ± 3.44 ng/mg by 30 days. Histologically, DCB treatments were associated with favorable tissue responses at both doses, consistent with device biocompatibility. Endothelialization was complete in both groups, and neointima was minimal. Arterial injury was rare and negligible, while inflammation was overall negligible to minimal. As shown in FIG. 11A, the DCB treatments had comparably benign histomorphometry scores following 1x or 3x inflations of the DCB. Endothelialization was complete in both groups (1x and 3x), neointima was minimal in both groups (1x and 3x), and adventitial fibrosis was minimal in the 3x group. Arterial injury was rare and negligible, while inflammation and luminal fibrin/thrombus were overall negligible to minimal. As shown in FIG. 11B, an artery from the 1x group appeared histologically within normal limits while an artery from the 3x group exhibited localized medial pallor/ degeneration and thin neointima. However, both arteries exhibited complete endothelialization, and no thrombosis, injury, necrosis or adventitial fibrosis was observable. Macroscopic, histologic and SEM findings indicated arterial patency and lack of thrombogenicity in both groups. A shown in FIG. 12, the SEM findings of both groups (1x and 3x) seven days after treatment using the DCB revealed ∼75% endothelialization, and near baseline levels of inflammation (leukocyte adherence score) and clotting (fibrin thrombus).

While about 75% of the PTX load was released from the balloons independent of the number of inflations per artery, the amount inserted in the wall and retained over time differed. Arteries in the clinical single dose group absorbed only 72.1±0.6 µg PTX, of which 97% was cleared with a half-life of 8 hours. Although one skilled in the art would expect, at most, a 3x retention for 3x inflation using three DCBs, or 216.3 µg PTX, the results of repeated treatments provided a synergistic increase in tissue retention. Surprisingly, three exposures produced 6.6-fold more PTX, twice the difference from dose alone (478±8.1 µg, p<0.0001) and what was deposited was cleared 1.6 times slower; 92% with a half-life of 13 hours versus 8 hours (p=0.0028).

Conclusions: Overall, treatment in peripheral arteries with DCB embodiments of this disclosure at 1x clinical dose or 3x safety margin dose, resulted in acceptable acute device performance, no adverse safety events, and pharmacokinetics similar to other PTX coated balloons. Tissue retention from embodiments of this disclosure benefits from repeat inflations but not due to increased dose exposure. Repeated dilation may prepare the artery for enhanced absorption and/or overcome restrictions of single dose retention.

EXAMPLE 2: The preclinical performance of an embodiment of a DCB was evaluated, where paclitaxel (PTX) was applied to an ePTFE porous surface of the balloon.

Methods: PTX coated balloons (5mm x 40mm and 6mm x 40mm ePTFE - W.L. Gore, Flagstaff AZ) with dose densities of 3.5 µg/mm² (labeled amount) or 3.3 µg/mm² (measured amount) of paclitaxel were inflated in peripheral arteries of Yorkshire swine. PTX coated balloons (Commercial) with dose densities of 3.5 µg/mm² (labeled amount) or 3.3 µg/mm² (measured amount) of paclitaxel were inflated in peripheral arteries of Yorkshire swine. Arteries received a clinical dose via a single treatment of inflation for 60 seconds. Animals were euthanized from 1h to 30 days post-treatment and subjected to comprehensive necropsies. The PTX coated balloons were collected for analysis of paclitaxel release, and treated arteries, along with other tissues, were collected for bioanalysis or processed for histologic and scanning electron microscopy (SEM) evaluation.

Additionally, PTX coated balloons (5mm x 40mm and 6mm x 40mm ePTFE - W.L. Gore, Flagstaff AZ) with dose densities of 3.5 µg/mm² were inflated and deflated at a benchtop. PTX coated balloons (Commercial) with dose densities of 3.5 µg/mm² were inflated and deflated at a benchtop. Particulates of the coating from each balloon were collected for analysis after the inflation/deflation.

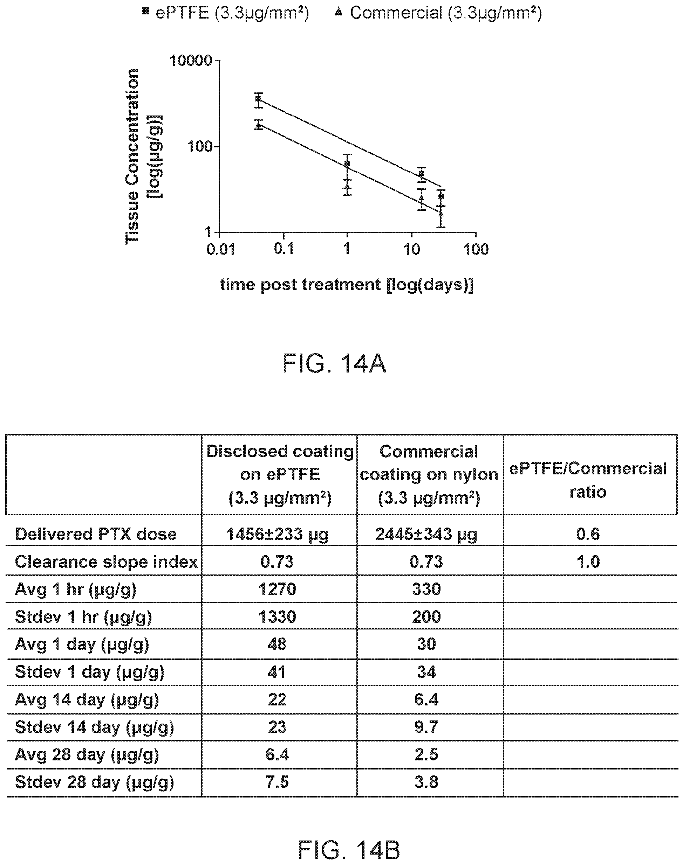

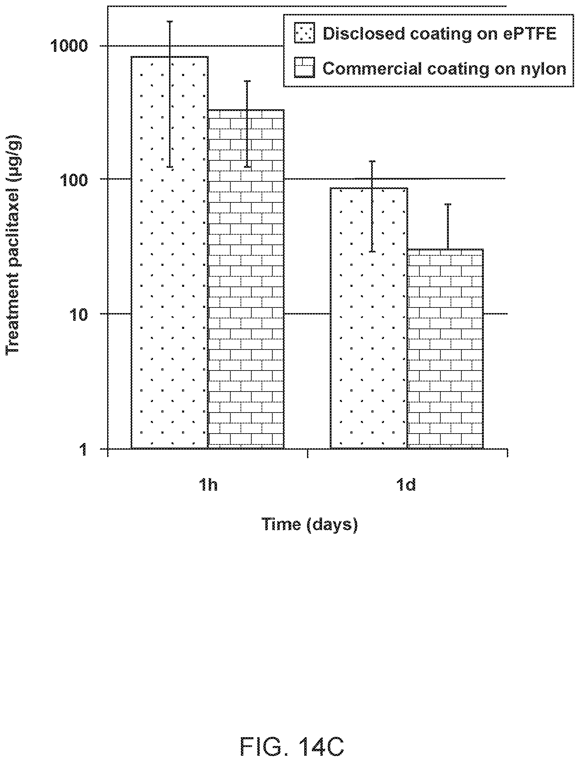

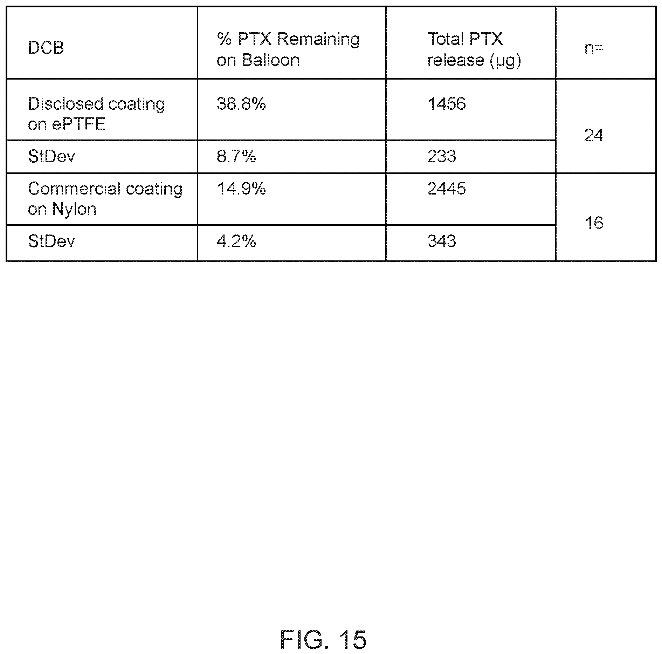

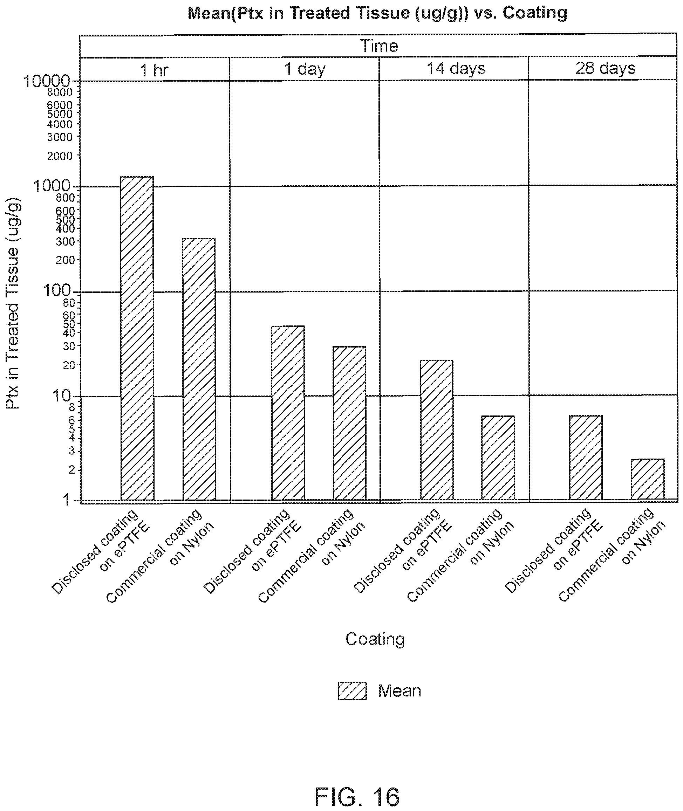

Results: As shown in FIGS. 14A, 14B, and 14C, FIG. 15, and FIG. 16 the PTX coated balloons according to embodiments of the disclosure (ePTFE - W.L. Gore, Flagstaff AZ) at a dosage density of 3.5 µg/mm² released 38.8% of a loaded dose amount, for example 1456 ± 233 µg of paclitaxel, and achieved a tissue concentration or dose amount of 1270 µg/g at one hour, and 48 µg/g at 1 day, 22 µg/g at 14 days, and 6.4 µg/g at 28 days respectively, and a clearance slope index of 0.73. The PTX coated balloons (Commercial) at a similar dosage density of 3.5 µg/mm², released 14.9% of a loaded dose amount, for example 2445 +/- 343 µg of paclitaxel, and achieved a tissue concentration or dose amount of 330 µg/g of paclitaxel at 1 hour, 30 µg/g at 1 day, 6.4 µg/g at 14 days, and 2.5 µg/g at 28 days respectively, and a clearance slope index of 0.73.

While the PTX coated balloons (Commercial) delivered a greater initial PTX dose compared to that of the PTX coated balloons according to embodiments of the disclosure (ePTFE - W.L. Gore, Flagstaff AZ) (an ePTFE/Commercial ratio of 0.6), the PTX coated balloons (ePTFE - W.L. Gore, Flagstaff AZ) were able to achieve a 3.8 fold greater maximum retainable dose at 1 hour compared to the PTX coated balloons (Commercial) (an ePTFE/Commercial ratio of 3.8) and a 1.6 fold greater maximum retainable dose at 1 day compared to the PTX coated balloons (Commercial) (an ePTFE/Commercial ratio of 1.6). Although one skilled in the art would expect that the PTX coated balloons (Commercial) delivering a higher initial dose of PTX over that of the PTX coated balloons (ePTFE - W.L. Gore, Flagstaff AZ) would also have a higher retainable dose at 1 hour or 1 day over that of the PTX coated balloons (ePTFE - W.L. Gore, Flagstaff AZ), the results of DCBs with a fluoropolymer surface and a microcrystalline surface coating morphology provided a synergistic increase in tissue retention.

Surprisingly, the fluoropolymer surface and a microcrystalline surface coating morphology of the PTX coated balloons (ePTFE - W.L. Gore, Flagstaff AZ) produced a 3.8-fold higher retainable drug delivery at 1 hour and a 1.6-fold higher retainable drug delivery at 1 day over that of the PTX coated balloons (Commercial). Moreover, the PTX coated balloons (ePTFE - W.L. Gore, Flagstaff AZ) showed no signs of particulation after inflation/deflation at a benchtop, whereas the PTX coated balloons (Commercial) demonstrated particulation.

Conclusions: Overall, treatment in peripheral arteries with DCB embodiments of this disclosure at a dose density of 3.5 µg/mm², resulted in acceptable acute device performance, no adverse safety events, and pharmacokinetics similar to other PTX coated balloons. Imaging of the balloon surfaces coated with similar paclitaxel doses (3.5 µg/mm2) revealed different morphologies. Tissue retention from embodiments of this disclosure benefits from the fluoropolymer surface and a microcrystalline surface coating morphology of the coated balloons but not due to increased dose exposure. The microcrystalline surface coating morphology in some manner may decrease particulation and increase tissue retention of the drug. The similar clearance rates may be attributable to similar tissue clearance mechanisms for both type of DCBs, which demonstrates the importance of early delivery and retention of the drug.

EXAMPLE 3: The preclinical performance of an embodiment of a DCB was evaluated, where paclitaxel (PTX) was applied a surface of the balloon.

Methods: PTX coated balloons comprising a fluoropolymer and a coating according to embodiments of the present disclosure (5mm x 40mm and 6 mm x 40 mm ePTFE - W.L. Gore, Flagstaff AZ) with dose densities of 4.1 µg/mm² (5mm x40 mm balloon) or 3.6 µg/mm² (6mm x40 mm balloon),were inflated for 60 seconds in peripheral arteries of 9 Yorkshire swine (target balloon-to-artery ratio between 1.05:1-1.2:1). PTX coated balloons comprising nylon and a coating according to embodiments of the present disclosure (5mm x 40mm and 6 mm x 40 mm nylon - W.L. Gore, Flagstaff AZ)with dose densities of 4.1 µg/mm² (5mm x40 mm balloon) or 3.6 µg/mm² (6mm x40 mm balloon) were inflated for 60 seconds in peripheral arteries of 9 Yorkshire swine (target balloon-to-artery ratio between 1.05:1-1.2:1). , Comparative (commercial) PTX coated commercial balloons comprising nylon and a commercial coating with a dose density of 3.5 µg/mm² were inflated for 60 seconds in peripheral arteries of 9 Yorkshire swine (target balloon-to-artery ratio between 1.05:1-1.2:1. Vessels were harvested at 1 hour, 24 hours, and 72 hours after treatment and were bisected, mounted and processed for scanning electron microscopy to quantify enface tissue coating. Paclitaxel amount in the harvested tissue was measured post imaging.

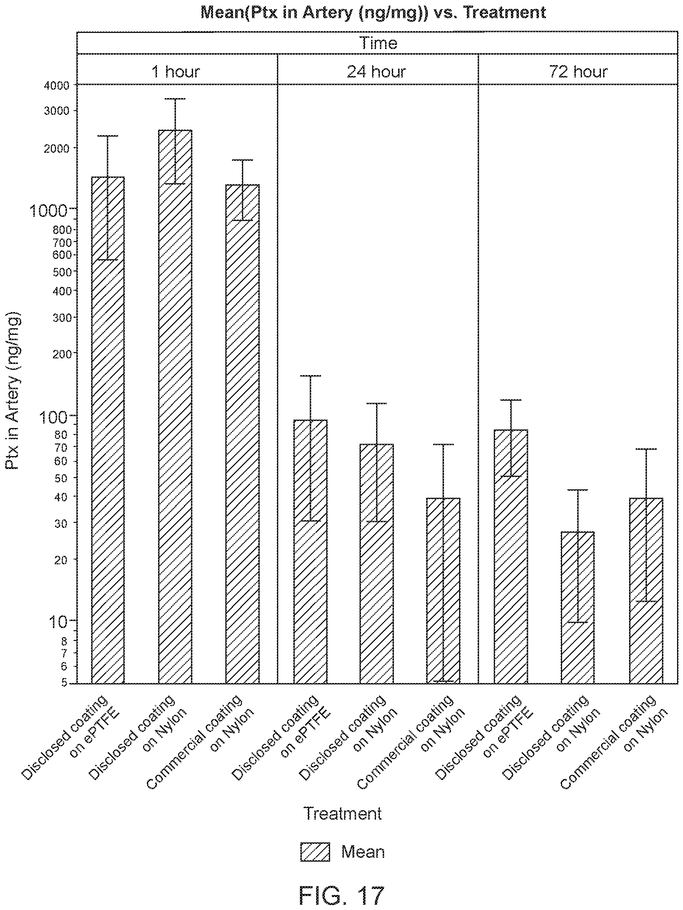

Results: DCB treatments were successful in peripheral vessels. As shown in FIG. 13 and FIG. 17, the PTX ePTFE balloons comprising a coating according to embodiments of the disclosure ("PTX coated ePTFE balloon"- W.L. Gore) released 77.1 % of a loaded dose, for example 2460 µg of paclitaxel, PTX nylon balloons comprising a coating according to embodiments of the disclosure ("PTX coated nylon balloon" - W.L. Gore) released 95.4%, for example 2780 µg of paclitaxel, and a PTX nylon balloons comprising a commercial coating ("PTX commercial nylon balloon") released 83.9%, for example 2350 µg of paclitaxel. As shown in FIG. 6A, PTX coated ePTFE balloons according to embodiments of the disclosure achieved a drug coating for an entire therapeutic site of the peripheral arteries that exhibited about 18.7% enface tissue coating at 1 hour post treatment and about 1.1% enface tissue coating at 1 day post treatment The PTX coated nylon balloons according to embodiments of the disclosure achieved a drug coating for an entire therapeutic site of the peripheral arteries that exhibited about 14.3% enface tissue coating at 1 hour post treatment and about 0.6% enface tissue coating at 1 day post treatment, whereas PTX commercial nylon balloons achieved a drug coating for an entire therapeutic site on the inner surface of a blood vessel that exhibited about 10.4% enface tissue coating at 1 hour post treatment and about 0.2% enface tissue coating at 1 day post treatment. As further shown in FIG. 17 PTX coated ePTFE balloons according to embodiments of the disclosure achieved tissue paclitaxel concentrations of 1450, 95, and 85 µg/g at 1 hour, 1 day, and 3 days, respectively, PTX coated nylon balloons according to embodiments of the disclosure achieved tissue paclitaxel concentrations of 2420, 73, and 27 µg/g at 1 hour, 1 day, and 3 days, respectively, and PTX commercial nylon balloons achieved tissue paclitaxel concentrations of 1300, 39, and 40 µg/g at 1 hour, 1 day, and 3 days, respectively.

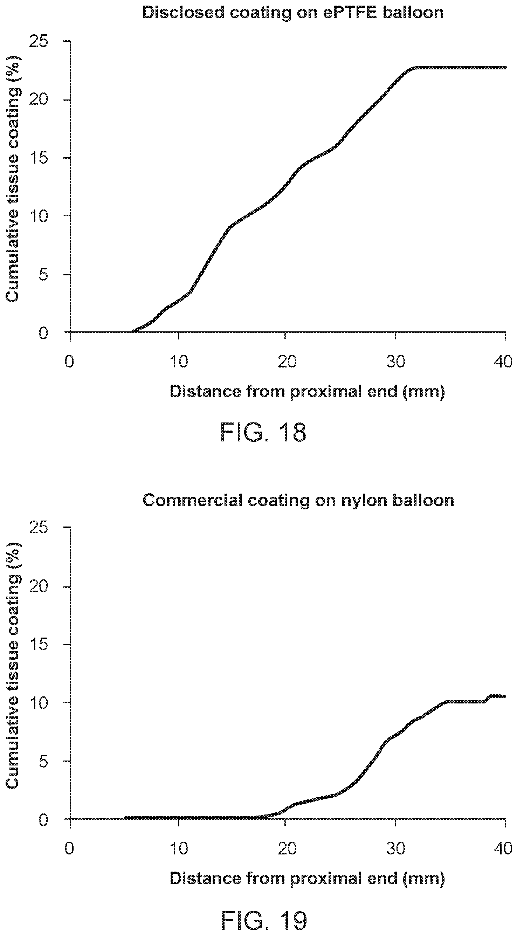

FIG. 18 depicts the enface tissue coating of the therapeutic site at 1 hour post treatment using the PTX coated ePTFE balloons according to embodiments of the disclosure on an ePTFE balloon. FIG. 18 depicts the enface tissue coating at various points along the length of the therapeutic site (40 mm in length). FIG. 19 depicts the enface tissue coating of the therapeutic site at 1 hour post treatment using a PTX commercial nylon balloons. FIG. 19 depicts the enface tissue coating at various points along the length of the therapeutic site (40 mm in length). FIG. 18 as compared to FIG. 19 demonstrates that the enface tissue coating using the PTX coated ePTFE balloons comprising a coating according to embodiments of the disclosure is improved as compared to the enface tissue coating using the PTX commercial nylon balloons comprising a commercial coating. For example, the enface tissue coating at 20 mm from the proximal end of the treatment site is between about 5% and about 25%, for example between about 10% and about 20%, for example approximately 13% for the PTX coated ePTFE balloons. Compared to between about 0% and about 10%, for example between about 0% and about 5%, or for example approximately 2% for the PTX commercial balloons. Moreover, FIGS. 18 and 19 demonstrate that the enface tissue coating for the PTX commercial balloons has an enface coverage percentage of between greater than about 10% along at least a portion of the treatment site, for example greater than about 15%, or greater than about 20%. In comparison, the PTX coated ePTFE balloons has an enface coverage percentage of between about 0% and about 14% along the length of the treatment site. Thus, the PTX coated ePTFE balloons demonstrate improved enface coating along the length of the treatment site, including an enface tissue coating of greater than 15% along at least a portion of the treatment site.

Conclusions: Overall, treatment in peripheral arteries with DCB embodiments of this disclosure resulted in acceptable acute device performance, no adverse safety events, and pharmacokinetics similar to other PTX coated balloons. Imaging of the balloon surfaces coated with similar paclitaxel doses revealed different morphologies, and imaging of treated surfaces of peripheral arteries of the Yorkshire swine revealed different coating efficiencies that correlated with the different paclitaxel delivery and retention. Tissue retention from embodiments of this disclosure benefits from the fluoropolymer surface and a microcrystalline surface coating morphology of the coated balloons but not due to increased dose exposure. While all balloons had similar tissue concentrations at 1 hour post treatment, the PTX ePTFE balloon (W.L. Gore) achieved approximately 2 to 3 times the tissue concentration of the PTX commercial nylon balloon and PTX coatednylon balloons according to embodiments of the disclosure , respectively, at 3 days.

EXAMPLE 4: The preclinical performance of a drug coating formulation prepared in accordance with some aspects discussed herein was evaluated for treating various types of ePTFE microstructures and Nylon.

Methods: An ePTFE precursor film (A) that was amorphously locked and generally made in accordance with U.S. Pat. No. 3,953,566 had the following properties: thickness of approximately 25 µm, mass per area of approximately 9 g/m², and a bubble point of approximately 14 kPa. An ePTFE precursor film (B) that was amorphously locked had the following properties: thickness of approximately 0.0017 inch, mass per area of approximately 6.23 gsm, and a bubble point of approximately 0.41 psi. An ePTFE precursor film (C) that was amorphously locked had the following properties: thickness of approximately 0.00025 inch, mass per area of approximately 2.66 gsm, and a bubble point of approximately 20.38 psi. Tubular members of the ePTFE precursor films were generally made in accordance with U.S. Patent Application Publication No. 2016/0106961. For example, in forming circumferentially wrapped tubular members, in some embodiments, the ePTFE precursor film material was cut into a wide sheet or tape, wherein the strongest direction was transverse to the length of the tape and the strongest direction was oriented axially in the formed balloon. In other circumferentially wrapped embodiments, the strongest direction was along the length of the sheet or tape such that the strongest direction was oriented circumferentially in the formed balloon. For forming helically wrapped tubular members, the precursor material was cut into a tape, wherein the strongest direction was along the length of the tape.

Additionally, PTX coated balloons (W.L. Gore, Flagstaff AZ) with dose densities of 3.5 µg/mm² (5x40 mm) were generally made from the tubular members of the ePTFE precursor films and Nylon in accordance with U.S. Patent Application Publication No. 2016/0106961. The PTX coated balloons were inflated in peripheral arteries of Yorkshire swine. Arteries received a clinical dose via a single treatment of inflation for 60 seconds. Animals were euthanized from 1h to 30 days post-treatment and subjected to comprehensive necropsies. The PTX coated balloons were collected for analysis of paclitaxel release, and treated arteries, along with other tissues, were collected for bioanalysis or processed for histologic and scanning electron microscopy (SEM) evaluation.

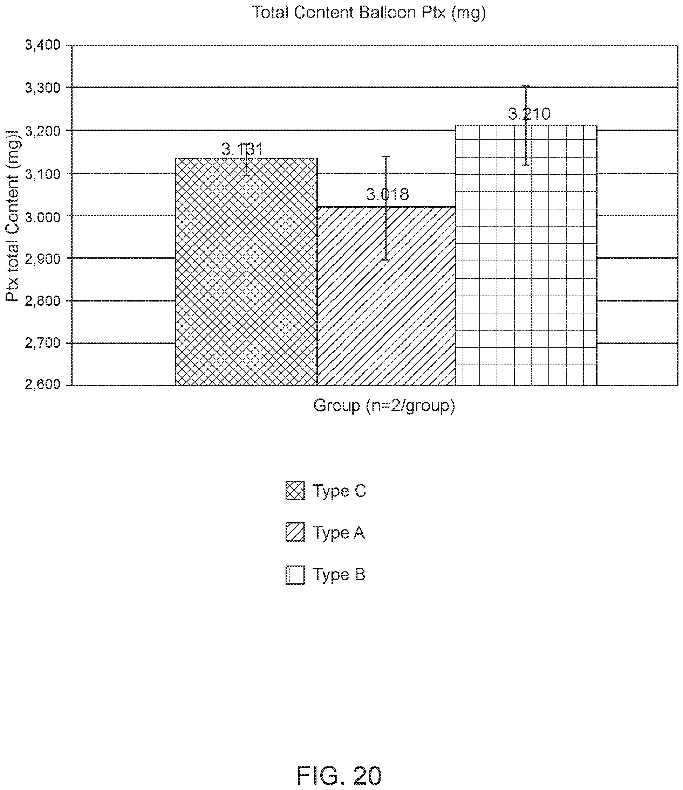

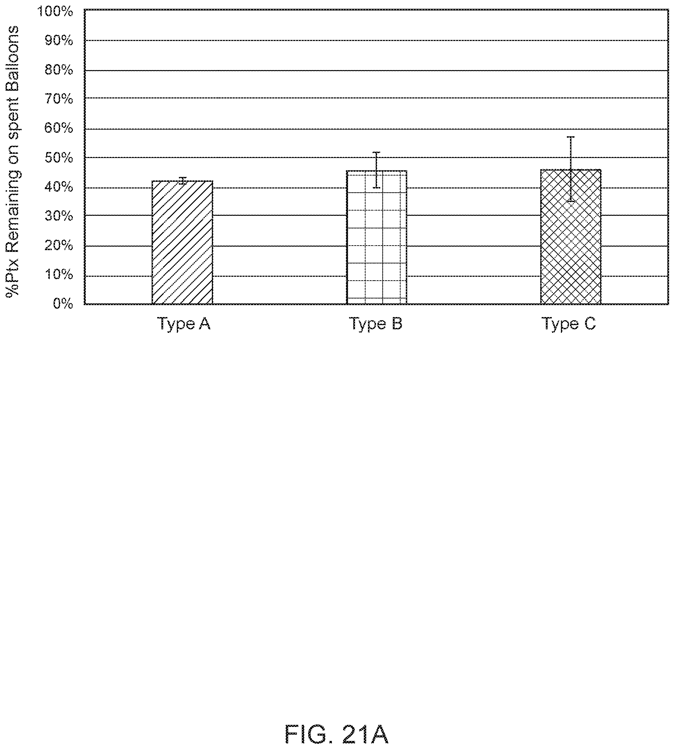

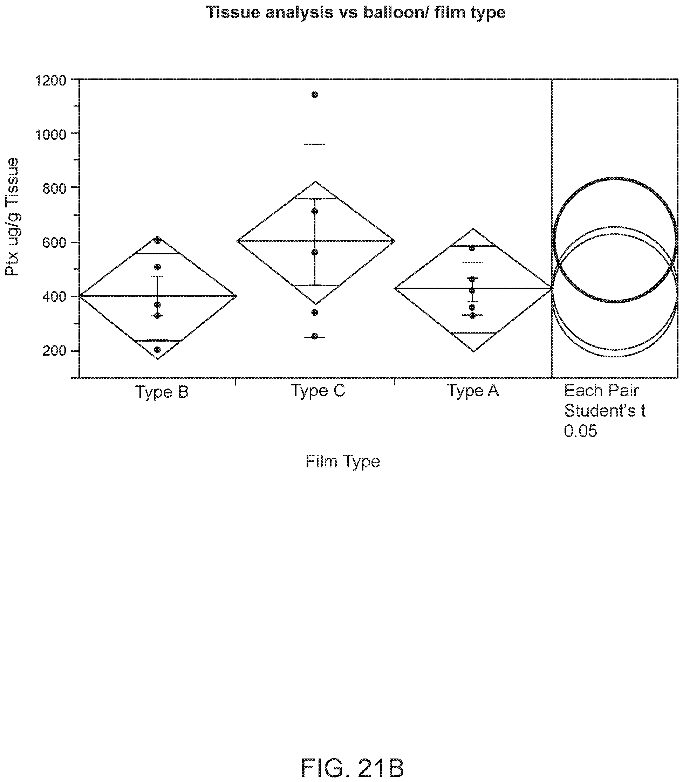

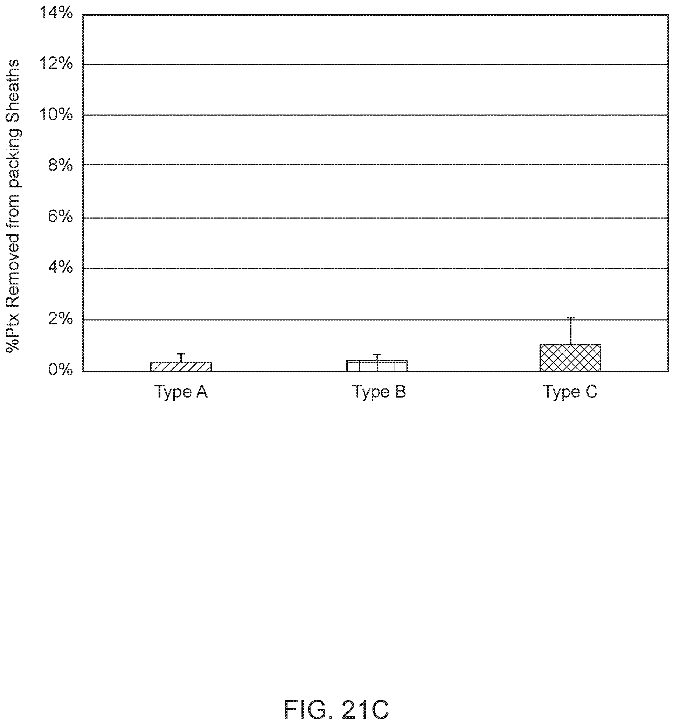

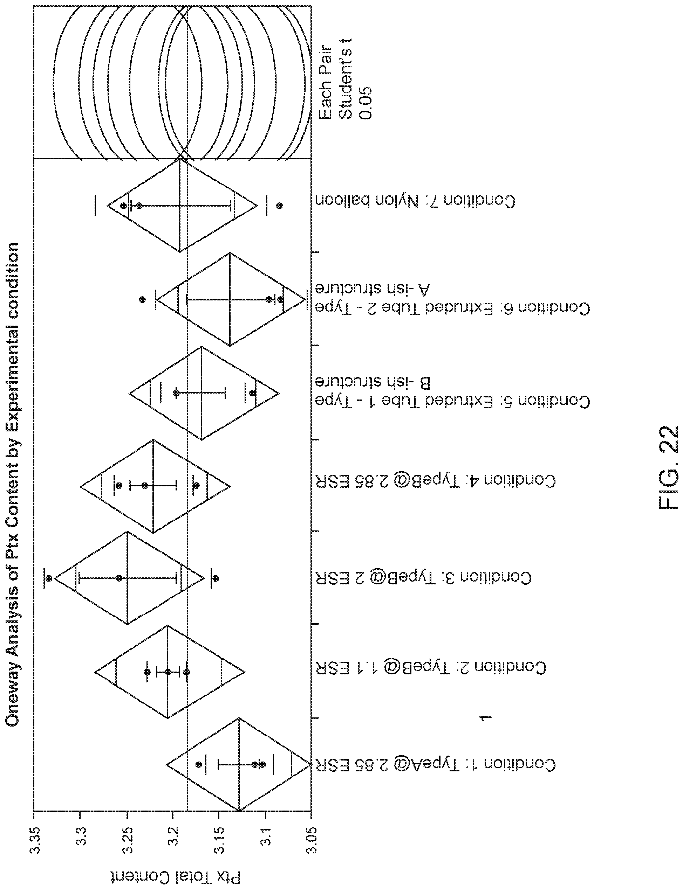

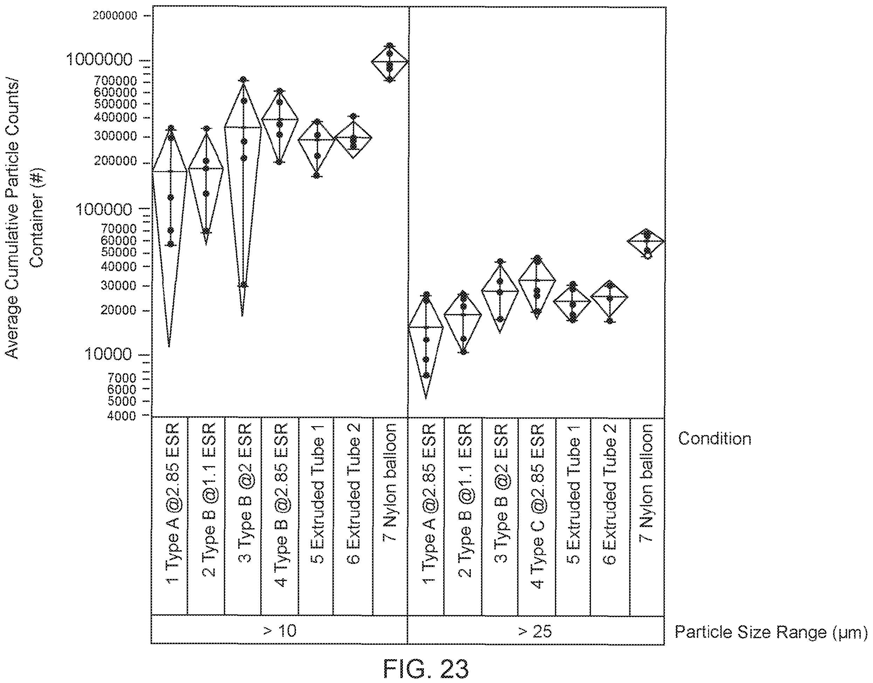

Results: As shown in FIG. 20, all three type of ePTFE (i.e., Film (A), (B), and (C) demonstrated a similar amount of drug on the surface, showing that wettability concerns with film change do not result in statistically different drug loads on the DCBs. As further shown in FIGS. 21A, 21B, and 21C, there is not statistical difference seen between the various types of ePTFE film (i.e., Film (A), (B), and (C) after acute tissue transfer. For example, in FIG. 21A, Film (A), (B), and (C) each comprised between 40% and 50% PTX remaining on balloons that had been inflated for treatment. FIG. 21B shows that similar amounts of PTX were transferred to the tissue after treatment. FIG. 21C shows the drug content on the packing sheath was checked to account for the whereabouts of all drug placed on each device for mass balance accounting, and demonstrated similar PTX amounts within the sheath for Film (A), (B), and (C). As further shown in FIG. 22, all three type of ePTFE (i.e., Film (A), (B), and (C) and Nylon demonstrated a similar amount of drug on the surface (specification is target load +/- 15% statistical difference seen is acceptable), showing that wettability concerns with film material change do not result in statistically different drug loads on the DCBs. As further shown in FIG. 23, there is not statistical difference seen in particulation during inflation/deflation between the various types of ePTFE film (i.e., Film (A), (B), and (C). However, the Nylon type DCBs did demonstrate a slightly greater degree of particulation.

Conclusions: The data shows that a range of ePTFE microstructures can be coated and used to deliver a drug such as paclitaxel to a treatment site. Additionally, the data suggests that Nylon DCBs, as compared to ePTFE DCBs, demonstrated a greater degree of particulation. However, overall, the data is conclusive that both a range of ePTFE microstructures and Nylon can be coated with formulations prepared in accordance with some aspects discussed herein and used to deliver a drug to a treatment site.

It should be understood that aspects of the invention and portions of various embodiments and various features recited above may be combined or interchanged either in whole or in part. In the foregoing descriptions of the various embodiments, those embodiments which refer to another embodiment may be appropriately combined with other embodiments as will be appreciated by the skilled artisan. Furthermore, the skilled artisan will appreciate that the foregoing description is by way of example only, and is not intended to limit the invention.

## Claims

1. A medical device comprising:
a balloon comprising an outer surface; and
a drug coating layer on the outer surface of the balloon,
wherein the drug coating layer comprises a plurality of microcrystals in a haystack orientation in which the majority of the plurality of microcrystals each have a major dimension length that is at least five times greater than the major dimension width of the microcrystal and the majority of said microcrystals are oriented at less than a 20 degree angle from the outer surface of the balloon, the microcrystals having random and a substantial absence of uniformity in placement on the outer surface of the balloon.

2. The medical device of claim 1, wherein the microcrystals have a random and a substantial absence of uniformity in angles from the outer surface of the balloon.

3. The medical device of claim 1, wherein the drug coating comprises paclitaxel.

4. The medical device of claim 1, wherein the outer surface of the balloon comprises a non-porous polymer; or wherein the outer surface of the balloon comprises a porous polymer.

5. The medical device of claim 1, wherein the balloon comprises a layered material, wherein the layered material comprises a polymer layer adhered to a fluoropolymer layer comprising a porous microstructure, wherein layers are in overlying relationship to each other and the fluoropolymer layer is an outermost layer; and optionally wherein the drug coating layer penetrates the outer surface of the balloon by an average penetration depth of from 2 to 10 µm.

6. A medical balloon comprising:
a thermoplastic polymeric layer defining an interior chamber;
a polymeric layer over at least a portion of the thermoplastic polymeric layer; and
a coating layer on at least a portion of the polymeric layer,
wherein the coating layer comprises a therapeutic agent and an excipient; and
wherein the coating layer comprises a plurality of microcrystals in a haystack orientation in which the majority of the plurality of microcrystals each have a major dimension length that is at least five times greater than the major dimension width of the microcrystal and the majority of said microcrystals are oriented at less than a 20 degree angle from the outer surface of the balloon, the microcrystals having random and a substantial absence of uniformity in placement on an outer surface of the polymeric layer.

7. The medical balloon of claim 6, wherein the polymeric layer is porous; or wherein the polymeric layer is non-porous.

8. The medical balloon of claim 6, wherein a majority of the microcrystals each have a major dimension length that is at least 10 times greater than a major dimension width of the microcrystal; or wherein the microcrystals have a random and a substantial absence of uniformity in angles from the outer surface of the polymeric layer, and a majority of the microcrystals project from the outer surface at an angle of 5° to 15°.

9. The medical balloon of claim 6, wherein the therapeutic agent comprises paclitaxel, docetaxel, protaxel, arsenic trioxide, thalidomide, atorvastatin, cerivastatin, fluvastatin, betamethasone diproprionate, dexamethasone 21-palmitate, sirolimus, everolimus, zotarolimus, biolimus or temsirolimus; or wherein the coating layer comprises the therapeutic agent and the excipient in a predetermined weight ratio of between 3:1 and 20:1.

10. The medical balloon of claim 6, wherein a majority of the microcrystals each have a major dimension length that is at least 13 or at least 15 times a major dimension width; or wherein a majority of the microcrystals each have a major dimension length that is between 12 µm and 22 µm, and the majority of the microcrystals each have a major dimension width that is between 0.5 µm and 2 µm.

11. The medical balloon of claim 6, wherein when the medical balloon is inflated in a vessel lumen for one minute, at least a portion of the coating layer transfers to at least a portion of the vessel lumen such that one hour after the inflation at least 14% of the portion of the vessel lumen is covered by the coating layer; or wherein when the medical balloon is inflated in a vessel lumen for one minute, at least a portion of the coating layer transfers to at least a portion of the vessel lumen such that one hour after the inflation at least 12% of a portion of a surface of the vessel lumen is covered by the coating layer.

12. The medical balloon of claim 6, wherein when the medical balloon is inflated in a vessel lumen for one minute, at least a portion of the coating layer uniformly transfers to the vessel lumen along a length of the vessel lumen.

13. The medical device of claim 1 comprising:
a) an elongate catheter shaft having a proximal section, a distal section, and an inflation lumen;
b) the balloon on the distal section of the shaft and comprising a balloon wall defining a chamber and comprising a layered material, wherein the layered material comprises a polymer layer adhered to a fluoropolymer layer comprising a porous microstructure, wherein layers are in overlying relationship to each other and the fluoropolymer layer is an outermost layer; and
c) the drug coating on the fluoropolymer layer.

14. The medical device of claim 13, wherein the drug coating comprises paclitaxel and an excipient; and optionally wherein the drug coating comprises paclitaxel and the excipient in a predetermined weight ratio of between 3:1 and 20:1

15. The medical device of claim 13, wherein a dose density of the drug coating is from 2.0 to 7.0 µg/mm²; or wherein the balloon is configured to release from 70% to 85% of a drug from the drug coating upon an inflation time of about 60 seconds; or wherein the microcrystals have a random and a substantial absence of uniformity in angles from the fluoropolymer layer, and a majority of the microcrystals project from the outer surface at an angle of 5° to 15°; or wherein the drug coating penetrates the fluoropolymer layer by an average penetration depth of from 2 to 10 µm.

16. The medical device of claim 6, wherein the balloon comprises a fluoropolymer surface and, upon an inflation time of about 60 seconds, the balloon is configured to release 68% to 83% of the therapeutic agent.

## Patentansprüche

1. Medizinische Vorrichtung, umfassend:
einen Ballon mit einer äußeren Oberfläche; und
eine Medikamentenbeschichtung auf der äußeren Oberfläche des Ballons;
wobei die Medikamentenbeschichtung eine Mehrzahl von Mikrokristallen in einer Heuhaufenausrichtung umfasst, wobei die Mehrheit der Mehrzahl von Mikrokristallen jeweils eine Hauptabmessungslänge aufweist, die mindestens fünfmal größer ist als die Hauptabmessungsbreite des Mikrokristalls, und wobei die Mehrheit der Mikrokristalle in einem Winkel von weniger als 20 Grad von der äußeren Oberfläche des Ballons ausgerichtet ist, wobei die Platzierung der Mikrokristalle auf der äußeren Oberfläche des Ballons willkürlich ist und im Wesentlichen keine Einheitlichkeit aufweist.

2. Medizinische Vorrichtung nach Anspruch 1, wobei die Mikrokristalle willkürliche und im Wesentlichen uneinheitliche Winkel von der äußeren Oberfläche des Ballons aufweisen.

3. Medizinische Vorrichtung nach Anspruch 1, wobei die Medikamentenbeschichtung Paclitaxel umfasst.

4. Medizinische Vorrichtung nach Anspruch 1, wobei die äußere Oberfläche des Ballons ein nicht poröses Polymer umfasst, oder wobei die äußere Oberfläche des Ballons ein poröses Polymer umfasst.

5. Medizinische Vorrichtung nach Anspruch 1, wobei der Ballon ein Schichtenmaterial umfasst, wobei das Schichtenmaterial eine Polymerschicht umfasst, die an einer Fluorpolymerschicht haftet, die eine poröse Mikrostruktur umfasst, wobei die Schichten in sich überlagerndem Verhältnis zueinander angeordnet sind, und wobei die Fluorpolymerschicht eine äußerste Schicht ist, und wobei die Medikamentenbeschichtung optional die äußere Oberfläche des Ballons mit einer durchschnittlichen Penetrationstiefe von 2 bis 10 µm penetriert.

6. Medizinischer Ballon, umfassend:
eine thermoplastische Polymerschicht, die eine innere Kammer definiert;
eine Polymerschicht über wenigstens einem Teil der thermoplastischen Polymerschicht; und
eine Beschichtung auf mindestens einem Teil der Polymerschicht;
wobei die Beschichtung ein Therapeutikum und einen Träger umfasst; und
wobei die Beschichtung eine Mehrzahl von Mikrokristallen in einer Heuhaufenausrichtung umfasst, wobei die Mehrheit der Mehrzahl von Mikrokristallen jeweils eine Hauptabmessungslänge aufweist, die mindestens fünfmal größer ist als die Hauptabmessungsbreite des Mikrokristalls, und wobei die Mehrheit der Mikrokristalle in einem Winkel von weniger als 20 Grad von der äußeren Oberfläche des Ballons ausgerichtet ist, wobei die Platzierung der Mikrokristalle auf einer äußeren Oberfläche der Polymerschicht willkürlich ist und im Wesentlichen keine Einheitlichkeit aufweist.

7. Medizinischer Ballon nach Anspruch 6, wobei die Polymerschicht porös ist; oder wobei die Polymerschicht nicht porös ist.

8. Medizinischer Ballon nach Anspruch 6, wobei die Mehrheit der Mikrokristalle jeweils eine Hauptabmessungslänge aufweisen, die mindestens 10-mal größer ist als eine Hauptabmessungsbreite des Mikrokristalls; oder wobei die Mikrokristalle willkürliche und im Wesentlichen uneinheitliche Winkel von der äußeren Oberfläche der Polymerschicht aufweisen, und wobei eine Mehrheit der Mikrokristalle von der äußeren Oberfläche in einem Winkel von 5° bis 15° vorstehen.

9. Medizinischer Ballon nach Anspruch 6, wobei das Therapeutikum Paclitaxel, Docetaxel, Protaxel, Arsenik, Thalidomid, Atorvastatin, Cerivastatin, Fluvastatin, Betamethasondiproprionat, Dexamethason-21-Palmitat, Sirolimus, Everolimus, Zotarolimus, Biolimus oder Temsirolimus umfasst; oder wobei die Beschichtung das Therapeutikum und den Träger in einem vorbestimmten Gewichtsverhältnis zwischen 3:1 und 20:1 umfasst.

10. Medizinischer Ballon nach Anspruch 6, wobei eine Mehrheit der Mikrokristalle jeweils eine Hauptabmessungslänge aufweisen, die mindestens 13- oder mindestens 15-mal einer Hauptabmessungsbreite entspricht; oder wobei die Mehrheit der Mikrokristalle jeweils eine Hauptabmessungslänge aufweisen, die zwischen 12 µm und 22 µm liegt, und wobei die Mehrheit der Mikrokristalle jeweils eine Hauptabmessungsbreite aufweisen, die zwischen 0,5 µm und 2 µm liegt.

11. Medizinischer Ballon nach Anspruch 6, wobei, wenn der medizinische Ballon in einem Gefäßlumen eine Minute lang deflatiert wird, mindestens ein Teil der Beschichtung auf mindestens einen Teil des Gefäßlumens übertragen wird, so dass eine Stunde nach der Inflation mindestens 14% des Teils des Gefäßlumens mit der Beschichtung überzogen ist; oder wobei, wenn der medizinische Ballon in einem Gefäßlumen eine Minute lang deflatiert wird, mindestens ein Teil der Beschichtung auf mindestens einen Teil des Gefäßlumens übertragen wird, so dass eine Stunde nach der Inflation mindestens 12% eines Teils einer Oberfläche des Gefäßlumens mit der Beschichtung überzogen ist.

12. Medizinischer Ballon nach Anspruch 6, wobei, wenn der medizinische Ballon in einem Gefäßlumen eine Minute lang deflatiert wird, mindestens ein Teil der Beschichtung gleichmäßig entlang der Länge des Gefäßlumens auf das Gefäßlumen übertragen wird.

13. Medizinische Vorrichtung nach Anspruch 1, umfassend:
a) einen länglichen Katheterschaft mit einem proximalen Abschnitt, einem distalen Abschnitt und einem Inflationslumen;
b) den Ballon an dem distalen Abschnitt des Schafts und umfassend eine Ballonwand, die eine Kammer definiert und ein Schichtenmaterial umfasst, wobei das Schichtenmaterial eine Polymerschicht umfasst, die an einer Fluorpolymerschicht haftet, die eine poröse Mikrostruktur umfasst, wobei die Schichten in sich überlagerndem Verhältnis zueinander angeordnet sind, und wobei die Fluorpolymerschicht eine äußerste Schicht ist; und
c) die Medikamentenbeschichtung auf der Fluorpolymerschicht.

14. Medizinische Vorrichtung nach Anspruch 13, wobei die Medikamentenbeschichtung Paclitaxel und einen Träger umfasst; und wobei die Medikamentenbeschichtung optional Paclitaxel und einen Träger in einem Gewichtsverhältnis zwischen 3:1 und 20:1 umfasst.

15. Medizinische Vorrichtung nach Anspruch 13, wobei eine Dosisdichte der Medikamentenbeschichtung zwischen 2,0 und 7,0 µg/mm² liegt; oder wobei der Ballon so gestaltet ist, dass er bei einer Inflationszeit von etwa 60 Sekunden 70% bis 85% eines Medikaments aus der Medikamentenbeschichtung abgibt; oder wobei die Mikrokristalle willkürliche und im Wesentlichen uneinheitliche Winkel von der Fluorpolymerschicht aufweisen; und wobei eine Mehrheit der Mikrokristalle von der äußeren Oberfläche in einem Winkel von 5° bis 15° vorstehen; oder wobei die Medikamentenbeschichtung die Fluorpolymerschicht mit einer durchschnittlichen Penetrationstiefe von 2 bis 10 µm penetriert.

16. Medizinische Vorrichtung nach Anspruch 6, wobei der Ballon eine Fluorpolymeroberfläche umfasst, und wobei der Ballon so gestaltet ist, dass er bei einer Inflationszeit von etwa 60 Sekunden 68% bis 83% des Therapeutikums abgibt.

## Revendications

1. Dispositif médical comprenant :
un ballonnet comprenant une surface extérieure ; et
une couche d'enrobage de médicament sur la surface extérieure du ballonnet,
la couche d'enrobage de médicament comprenant une pluralité de microcristaux dans une orientation de botte de foin dans laquelle la majorité de la pluralité de microcristaux ont chacun une longueur de dimension majeure qui est au moins cinq fois plus grande que la largeur de dimension majeure du microcristal et la majorité desdits microcristaux sont orientés à moins d'un angle de 20 degrés par rapport à la surface extérieure du ballonnet, les microcristaux ayant une absence aléatoire et substantielle d'uniformité dans le placement sur la surface extérieure du ballonnet.

2. Dispositif médical selon la revendication 1, les microcristaux ayant une absence d'uniformité aléatoire et substantielle des angles par rapport à la surface extérieure du ballonnet.

3. Dispositif médical selon la revendication 1, l'enrobage de médicament comprenant du paclitaxel.

4. Dispositif médical selon la revendication 1, la surface extérieure du ballonnet comprenant un polymère non poreux ; ou la surface extérieure du ballonnet comprenant un polymère poreux.

5. Dispositif médical selon la revendication 1, le ballonnet comprenant un matériau en couches, le matériau en couches comprenant une couche de polymère adhérant à une couche de fluoropolymère comprenant une microstructure poreuse, les couches étant en relation de superposition les unes par rapport aux autres et la couche de fluoropolymère étant une couche la plus externe ; et éventuellement la couche d'enrobage de médicament pénétrant la surface extérieure du ballonnet par une profondeur de pénétration moyenne comprise entre 2 et 10 µm.

6. Ballonnet médical, comprenant :
une couche de polymère thermoplastique définissant une chambre intérieure ;
une couche de polymère sur au moins une partie de la couche de polymère thermoplastique ; et
une couche d'enrobage sur au moins une partie de la couche de polymère,
la couche d'enrobage comprenant un agent thérapeutique et un excipient ; et
la couche d'enrobage comprenant une pluralité de microcristaux dans une orientation de botte de foin dans laquelle la majorité de la pluralité de microcristaux ont chacun une longueur de dimension majeure qui est au moins cinq fois plus grande que la largeur de dimension majeure du microcristal et la majorité desdits microcristaux sont orientés à moins d'un angle de 20 degrés par rapport à la surface extérieure du ballonnet, les microcristaux ayant une absence aléatoire et substantielle d'uniformité dans le placement dans le placement sur la surface extérieure de la couche de polymère.

7. Ballonnet médical selon la revendication 6, la couche de polymère étant poreuse ; ou la couche de polymère étant non poreuse.

8. Ballonnet médical selon la revendication 6, une majorité des microcristaux ayant chacun une longueur de dimension majeure qui est au moins 10 fois supérieure à une largeur de dimension majeure du microcristal ; ou les microcristaux ayant une absence aléatoire et substantielle d'uniformité dans les angles par rapport à la surface extérieure de la couche de polymère, et une majorité des microcristaux faisant saillie par rapport à la surface extérieure selon un angle compris entre 5° et 15°.

9. Ballonnet médical selon la revendication 6, l'agent thérapeutique comprenant le paclitaxel, le docétaxel, le protaxel, le trioxyde d'arsenic, la thalidomide, l'atorvastatine, la cérivastatine, la fluvastatine, le diproprionate de bétaméthasone, le 21-palmitate de dexaméthasone, le sirolimus, l'évérolimus, le zotarolimus, le biolimus ou le temsirolimus ; ou la couche d'enrobage comprenant l'agent thérapeutique et l'excipient dans un rapport pondéral prédéfini compris entre 3:1 et 20:1.

10. Ballonnet médical selon la revendication 6, une majorité des microcristaux ayant chacun une longueur de dimension majeure qui est au moins 13 ou au moins 15 fois une largeur de dimension majeure ; ou une majorité des microcristaux ayant chacun une longueur de dimension majeure qui est comprise entre 12 µm et 22 µm, et la majorité des microcristaux ayant chacun une largeur de dimension majeure qui est comprise entre 0,5 µm et 2 µm.

11. Ballonnet médical selon la revendication 6, lorsque le ballonnet médical est gonflé dans une lumière de vaisseau pendant une minute, au moins une partie de la couche d'enrobage se transférant à au moins une partie de la lumière de vaisseau de sorte qu'une heure après le gonflement, au moins 14 % de la partie de la lumière de vaisseau est couverte par la couche d'enrobage ; ou, lorsque le ballonnet médical est gonflé dans une lumière de vaisseau pendant une minute, au moins une partie de la couche d'enrobage se transférant sur au moins une partie de la lumière de vaisseau de sorte qu'une heure après le gonflage, au moins 12 % d'une partie de la surface de la lumière de vaisseau est recouverte par la couche d'enrobage.

12. Ballonnet médical selon la revendication 6, lorsque le ballonnet médical est gonflé dans une lumière de vaisseau pendant une minute, au moins une partie de la couche d'enrobage se transférant uniformément dans la lumière de vaisseau sur une longueur de la lumière de vaisseau.

13. Dispositif médical selon la revendication 1 comprenant :
a) une tige de cathéter allongée ayant une section proximale, une section distale et une lumière de gonflage ;
b) le ballonnet sur la section distale de la tige et comprenant une paroi de ballonnet définissant une chambre et comprenant un matériau stratifié, le matériau stratifié comprenant une couche de polymère adhérant à une couche de fluoropolymère comprenant une microstructure poreuse, les couches étant en relation de recouvrement les unes par rapport aux autres et la couche de fluoropolymère étant une couche la plus externe ; et
c) l'enrobage de médicament sur la couche de fluoropolymère.

14. Dispositif médical selon la revendication 13, l'enrobage de médicament comprenant du paclitaxel et un excipient ; et éventuellement l'enrobage de médicament comprenant du paclitaxel et l'excipient dans un rapport pondéral prédéfini compris entre 3:1 et 20:1.

15. Dispositif médical selon la revendication 13, la densité de dose de l'enrobage de médicament étant comprise entre 2,0 et 7,0 µg/mm² ; ou le ballonnet étant conçu pour libérer de 70 % à 85 % d'un médicament à partir de l'enrobage de médicament après un temps de gonflage d'environ 60 secondes ; ou les microcristaux ayant une absence aléatoire et substantielle d'uniformité dans les angles par rapport à la couche de fluoropolymère, et une majorité des microcristaux faisant saillie de la surface extérieure à un angle compris entre 5° et 15° ; ou l'enrobage de médicament pénétrant dans la couche de fluoropolymère avec une profondeur de pénétration moyenne comprise entre 2 et 10 µm.

16. Dispositif médical selon la revendication 6, le ballonnet comprenant une surface de fluoropolymère et, après un temps de gonflage d'environ 60 secondes, le ballonnet étant conçu pour libérer de 68 % à 83 % de l'agent thérapeutique.
